(19) Europäisches Patentamt
European Patent Office
Office européen des brevets

(11) Publication number: **0 123 504 B1**

(12) **EUROPEAN PATENT SPECIFICATION**

(45) Date of publication of patent specification: **20.09.89**

(21) Application number: **84302615.4**

(22) Date of filing: **17.04.84**

(51) Int. Cl.$^4$: **C 07 F 5/00,** A 61 K 49/02, C 07 C 131/00, C 07 D 295/12 // C07D295/14, C07C103/147, C07C87/18, C07C101/24

(54) **Complexes of technetium-99m with alkylene amine oximes.**

(30) Priority: **25.04.83 US 488184**

(43) Date of publication of application:
**31.10.84 Bulletin 84/44**

(45) Publication of the grant of the patent:
**20.09.89 Bulletin 89/38**

(84) Designated Contracting States:
**BE DE FR GB IT NL SE**

(56) References cited:
**EP-A-0 038 756**

**INORGANIC CHEMISTRY, vol. 12, no.11, 1973, pages 2631-2635; H. GOFF et al.: "Kinetic and thermodynamic studies on a labile trans-dinitrocobalt(III) complex"**

**THE JOURNAL OF NUCLEAR MEDICINE, vol. 20, no. 6, 1979, pages 641-642, New York, US; D.E. TROUTNER et al.: "Complexes of Tc-99m with cyclam"**

(73) Proprietor: **AMERSHAM INTERNATIONAL plc
White Lion Road
Amersham Buckinghamshire, HP7 9LL (GB)**

(72) Inventor: **Troutner, David Elliott
402, Edgewood Avenue
Columbia Missouri 65201 (US)**
Inventor: **Volkert, Wynn Arthur
2203, Garden Drive
Columbia Missouri 65201 (US)**

(74) Representative: **Pennant, Pyers et al
Stevens, Hewlett & Perkins 5 Quality Court
Chancery Lane
London, WC2A 1HZ (GB)**

(56) References cited:
**THE JOURNAL OF NUCLEAR MEDICINE, vol. 24, no. 5, May 1983, page P10, New York, US; D.E. TROUTNER et al.: "A tetradentate amine oxime complex of Tc-99m"**

**EP 0 123 504 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technetium-99m (Tc-99m) is the favoured radionuclide for organ imaging and other forms of *in vivo* diagnosis. Complexes of Tc-99m have been used for investigating most parts of the body.

This invention relates to complexes of technetium, e.g. technetium-99m complexes useful as diagnostic pharmaceuticals, and in particular to complexes which are capable of crossing the blood-brain barrier and being retained in the brain for a time to permit diagnosis.

Tc-99m is obtained from a technetium generator as pertechnetate ion $TcO_4^-$, with technetium in the 7+ valency, generally in saline solution. Since pertechnetate does not readily form complexes, a usual technique is to mix the pertechnetate with a suitable ligand under reducing conditions, whereby the technetium is reduced to a lower valency, generally 3+, 4+ or 5+ in which it forms the desired complex. A problem is that these complexes almost always carry an overall charge; all Tc-99m complexes so far approved by the FDA for routine use in nuclear medicine in USA have overall positive or negative charges. It is known that charged species will not readily pass through the blood-brain barrier (BBB), and so cannot easily be introduced into the brain.

Compounds labelled with short-lived positron emitters (e.g. C-11, 0-15 and F-18) have been used to successfully delineate regional cerebral blood flow (rCBF) in normal and diseased brain tissue using Positron Emission Transaxial Tomography in a limited number of Nuclear Medicine laboratories. The costs to produce these compounds (including an on-site medical cyclotron) are prohibitive for wide spread use. If we are to witness the performance of such studies in the daily practice of medicine, the development of agents capable of rCBF assessment but labelled with more available and less costly radionuclides emitting a single photon (i.e. a gamma-ray), such as to Tc-99m is highly desirable.

Two types of single photon emitting diagnostic agents have been used to assess brain blood flow patterns in humans. The first is best exemplified by Xe-133, an inert noble gas, that passively diffuses across the BBB and clears the tissue at a rate proportional to the blood flow through that tissue. The pattern of rCBF is determined by following the rate of Xe-133 clearance as a function of time at different sites of the brain using either a specialized Single Photon Emission Computed Tomographic (SPECT) instrument or a commercial multidetector system. The second class of compounds include those that passively diffuse across the BBB (with a high extraction efficiency) into the brain and become trapped in the brain tissue. The trapping allows time for determination of rCBF patterns by more conventional SPECT imaging devices. The two most widely used single photon brain perfusion agents of this latter type are:

I-123-N,N,N'-trimethyl-N'-(2-OH-3-methyl-5-iodobenzyl)-1,3-propanediamine, (I-123-HIPDM) and I-123-Iosoamphetamine, (I-123-IMP). Because Tc-99m has superior imaging properties and is more readily available and less expensive, any new Tc-99m labelled compounds with similar or even better capabilities for brain uptake (and/or washout) as Xe-133, I-123-IMP or I-123-HIPDM would find widespread clinical applicability.

Besides rCBF studies these neutral-lipophilic Tc-99m-chelates would also be desirable in imaging the lungs. I-123-iodoantipyrene is a hydrophobic compound that distributes following injection throughout the lung water and has been used to assess extravascular lung water (EVLW) in normal and disease states. This agent passively diffuses into the lung parenchyma and is washed out by pulmonary blood flow. Lung imaging has also been performed using I-123-IMP. I-123-IMP is taken up by lung tissue and slowly released, presumably due to its binding to intracellular low specificity, — high capacity endothelial amine receptors. Clearly, Tc-99m agents that exhibit properties similar to I-123-iodoantipyrene would have value for measuring regional EVLW imaging (particularly in patients with the acute respiratory distress syndrome). Likewise, a Tc-99m-compound that binds to amine receptors in the lung would be useful for performing metabolic lung imaging.

Imaging of heart muscle in patients with myocardial damage using fatty acids or fatty acid analogues labelled with positron emitters or I-123 has shown promise as a diagnostic tool. As indicated earlier, the high cost of producing compounds labelled with positron emitters precludes their widespread availability in the foreseeable future.

Under normoxic conditions the energy requirements of heart muscle are met by oxidation of fatty acids and, therefore, the extraction of free fatty acids by the normal myocardium is high. In areas of heart muscle damage where local $pO_2$ is decreased (eq. ischemia) fatty acid oxidation (i.e., beta-oxidation) and free fatty acid uptake is decreased. Regional fatty acid metabolism could be measured by determining the rate of clearance of the labelled fatty acid from the myocardium. Alternatively, a fatty acid analogue that enters the metabolic pathway, undergoes partial metabolism, and whose radioactive label is trapped within the myocardium would also reflect regional metabolic activity (i.e., similar to 18-F-fluorodeoxyglucose for brain metabolic images. The localized trapped activity can then be imaged. Fatty acid analogues labelled in various manners with I-123 are taken up by normal heart muscle and depending upon the structure of the specific compound will either clear rapidly following intracellular beta-oxidation (similar to C-11 labelled fatty acids) or can be structurally modified so they will be trapped by the myocardial tissue. I-123 can be attached directly to the omega-end (i.e., the end opposite the carboxylic acid group) of the alkyl chain or attached to the omega-end of the alkyl chain by means of a I-123-phenyl group. Both types of derivatives have been successfully used as myocardial imaging agents. The fact that good myocardial uptake is observed with fatty acid analogues where the bulk-hydrophobic I-123-phenyl

group is attached to the omega-end indicates that other lipophilic groups similarly attached will have little effect on their extraction by normal heart muscle. Karesh, et al., (J. Pharm. Chem., 66, 225, 1977) and Schneider et al., (J. Labelled Compounds and Radiopharmaceuticals, 19, 1326—1327, 1982) failed in their attempts to produce a Tc-99m-fatty acid analogue that localizes in heart muscle. Both investigators attached ligands to the omega-ends of fatty acid analogues that formed negatively charged Tc-99m-chelates and concluded that the charged chelate at the end of the alkyl chain prevented their intracellular transport. It is expected that a neutral-hydrophobic Tc-99m-chelate attached to the omega-end of fatty acid analogues will not prevent normal myocardial cell uptake (viz., these types of compounds would be structurally similar to the I-123-phenyl-omega-fatty acid analogues).

At least three neutral Tc-99m-complexes have been prepared and reported. See, Burns, et al., *J. Nucl. Med.,* 20, 641, 1979; Kramer, et al., J. Labelled Compounds and Radiopharmaceuticals, 19, 1598—1599, 1982; Yokoyama, et al., *J. Nucl. Med., 17,* 816—19 (1976). None of these Tc-99m complexes have been established for routine diagnostic use. These neutral ligands have not met all of the needed requirements; for example, the neutral complexes of Yokoyama, et al., are extremely difficult to derivatize and use —SH groups for complexation with Tc-99m as does the neutral complex of Burns et al. Ligands utilizing —SH groups for chelation of Tc-99m form stable complexes but may be difficult to store. The neutral chelate of Kramer et al., does not have desirable stability characteristics. As far as known, none of these ligands show a high extraction efficiency by the brain.

Ligands that employ only N-atoms for chelation of Tc-99m have been shown to readily form complexes in aqueous media. Tetraaza ligands and in particular macrocyclic tetraaza ligands, form very stable Tc-99m complexes. See, Troutner, et al., *J. Nucl. Med.,* 21, 443—448 (1980), which describes the complexing of Tc-99m with the macrocyclic tetraaza ligand, cyclam. As far as is known, the only published study showing full characterization of such a macrocyclic ligand complex of Tc was published in 1981 by Zuckman, et al., *Inorg. Chem.,* 20, 2386—2389. The complex with cyclam (1,4,8,11-tetraazacyclotetradecane) under reducing conditions was shown to produce a $TcO_2^{+1}$ core, and a resulting complex had a charge of +1.

As far as is known, the ligand specifically referred to in this disclosure as propylene amine oxime (PnAO) has not been employed to prepare a complex with Tc-99m. This ligand is 4,8-diaza-3,3,9,9-tetramethylundecane-2,10-dione bisoxime and is henceforth referred to as PnAO. Methods for preparing PnAO are well known as is its use in complexing metal ions. See, for example, Vassian, et al., (Inorg. Chem., 6, 2043—2046, 1967). Complexation of PnAO to metal ions is by the 4 N-atoms and results in the ligand forming a cyclical structure around the chelated metal (Murmann et al, Inorg. Chem., 12, 2625—2631, 1973). The structural formula of PnAO is:

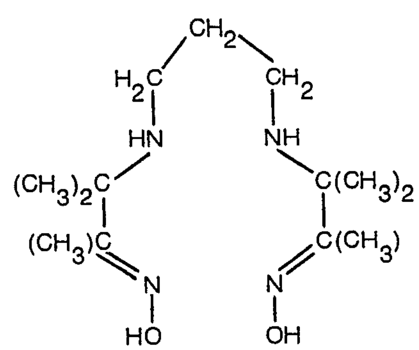

The corresponding ethylene amine oxime (EnAO) is 4,7-diaza-3,3,8,8-tetramethyldecane-2,9-dione bisoxime.

## Summary of the Invention

This invention is based in part on the discovery that technetium-99m can be complexed (by reduction of Tc-99m-pertechnetate in aqueous media) to propyleneamine oxime, to produce a stable lipophilic complex. This complex and derivatives will make useful diagnostic imaging agents. This appears to be a general complexation reaction with this type of amine oxime ligand since another form of the basic tetradentate amine oxime ligand, ethyleneamine oxime (EnAO) also forms a neutral-hydrophobic Tc-99m chelate in aqueous solution. The complexes have a zero net charge and are easily derivatizable. Tc-99m EnAO and PnAO are capable of crossing the blood-brain-barrier, passing through cell walls and being taken up efficiently by lung tissue, thereby extending their range of usefulness as radiopharmaceuticals. Further, the complexes are sufficiently stable, even in the presence of oxygen, to permit their preparation, storage and parenteral administration for imaging. The macrocyclic PnAO ring is closed during the formation of the complex by an O—H—O bond which serves to complete the ring and control the charge on the complex.

The structure of Tc-99 PnAO has been determined to be as shown in formula (3) below, in which $R^1$, $R^4$ and $R^5$ are methyl and $R^2$ and $R^3$ are hydrogen. It is expected that the structures of Tc-99 EnAO, of analogues of Tc-99 PnAO and of Tc-99 EnAO, and of the corresponding Tc-99m complexes are essentially as shown in formula (2) below. As will be noted, the technetium core is in the form of $TcO^{3+}$.

The statement that the technitium-99m complex of this invention has a core with zero net charge needs some explanation. The core of the complex, has no overall charge, in contrast to other similar Tc-99m complexes. But the attachment of charged or ionizable groups to the carbon atoms shown will result in the complex as a whole having a non-zero charge, at least under some conditions. Such complexes, having non-zero overall charge by virtue of charged or ionizable groups attached to the core, are of considerable interest for localizing in particular regions of the body, and are included within the scope of this invention. The term "zero net charge" thus refers to the macrocyclic core structure and ignores possible charged or ionizable substituent groups. Such designing of molecules is standard practice in research laboratories nowadays. The attachment of suitable groups to cause the complex to become located in a particular region or organ, such as the brain, of a mammal to which the complex is administered, will require no more than routine development work in the light of the common general knowledge in the field taken with the advice given in the specification. Complexes of isotopes of technetium other than Tc-99m are useful to determine the chemical properties of the complexes.

The present invention provides a lipophilic macrocyclic complex of Technetium-99m useful as a diagnostic radiopharmaceutical which can be formed by complexing in aqueous solution Tc-99m pertechnetate under reducing conditions with an alkylene amine oxime containing 2 or 3 carbon atoms in the alkylene group, which group is unsubstituted or substituted, the complex having a core with a zero net charge, containing an O—H—O ring closure bond, and being sufficiently stable for parenteral administration and imaging by scintillation scanning, any alkylene substituents present being of the kind useful for adapting radionuclide ligands for body imaging applications, which complex has the formula

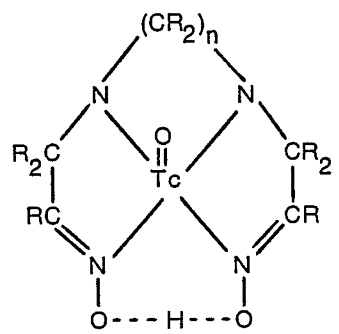

(2)

where n is 2 or 3, and the groups R may be the same or different and each is hydrogen, hydrocarbon of up to 22 carbon atoms which may be alkyl, alkenyl, alkaryl, aralkyl or aryl, primary secondary or tertiary amine, primary, secondary or tertiary amide, carboxylic acid or carboxylic acid ester, hydroxyl or alkoxyl, or an acceptor-acid group, and may be substituted or unsubstituted.

Preferred complexes have the formula

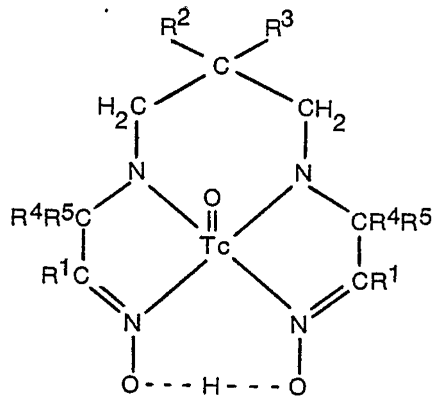

(3)

where each $R^1$, $R^4$ and $R^5$ is hydrogen or C1 to C12 alkyl, and each of $R^2$ and $R^3$ is hydrogen, hydroxyl, C1 to C12 alkoxyl, as up to C22 hydrocarbon which may be alkyl, alkenyl, alkaryl, aralkyl, or aryl, or tertiary amine with 1 to 20 carbon atoms, or $R^2$ and $R^3$ may form, together with the carbon atom to which they are attached, a cycloaliphatic group which may be amine substituted. Preferably, each of $R^2$ and $R^3$ is hydrogen or C1 to C4 alkyl.

As demonstrated below, complexes preferred for brain imaging have partition coefficients between lipid and water and molecular weights such that *in vivo* they are capable of diffusing across the blood brain barrier and of being retained in the brain for a time sufficient to be used for diagnostic purposes.

The invention also includes a method of body imaging which comprises administering to a mammal a complex of technetium-99m as defined above whereby the complex becomes localised in one or more regions of the mammal, and observing radiation emitted from the one or more regions.

The invention also includes a method of forming a complex of technetium as defined above by the steps of

a) complexing Tc-99m pertechnetate under reducing conditions with a complexing agent for technetium selected from alkylene amine oximes containing 2 or 3 carbon atoms in the alkylene group, which group is substituted or unsubstituted, any substituents present being of the kind useful for adapting radionuclide ligands for body imaging applications, and other weaker complexing agents for technetium,

b) if another weaker complexing agent for technetium was used in step a), reacting the resulting complex with an alkylene amine oxime as defined in a) under conditions to promote ligand exchange,

whereby there is formed the complex having a zero charge, containing an O—H—O ring closure bond, and being sufficiently stable for parenteral administration and imaging by scintillation scanning.

## Detailed Description

The propylene amine oxime ligand starting material for preparing the complexes of this invention can be prepared by known synthetic procedures. For example, PnAO (as well as EnAO) can be synthesized by the method of Vassian et al. Briefly described, this synthesis involves the reaction of 1,3-propanediamine (1,2-ethylenediamine) with the chloro-oxime reagent.

$$2\ CH_3\text{-}C(CH_3)Cl\text{-}C(CH_3)\text{=}NOH$$

$$+$$

$$CH_2(NH_2)\text{-}CH_2\text{-}CH_2(NH_2)$$

$$\downarrow$$

$$HON\text{=}C(CH_3)\text{-}C(CH_3)_2\text{-}NH.CH_2.CH_2.CH_2.NH\text{-}C(CH_3)_2\text{-}C(CH_3)\text{=}NOH$$

The same reaction sequence can be followed when the chloro-oxime reagent has the general structure

$$Cl—CR^4R^5—CR^1\text{=}NOH$$

when $R^4$, $R^5$ and $R^1$ are alkyl. Such reagents can be prepared by reacting NOCl with the appropriate alkene as follows:

$$CR^4R^5 = CR^1H + NOCl \rightarrow Cl—CR^4R^5 - CR^1 = NOH.$$

When $R^4$ and/or $R^5$ is hydrogen, another preparative route is preferred:

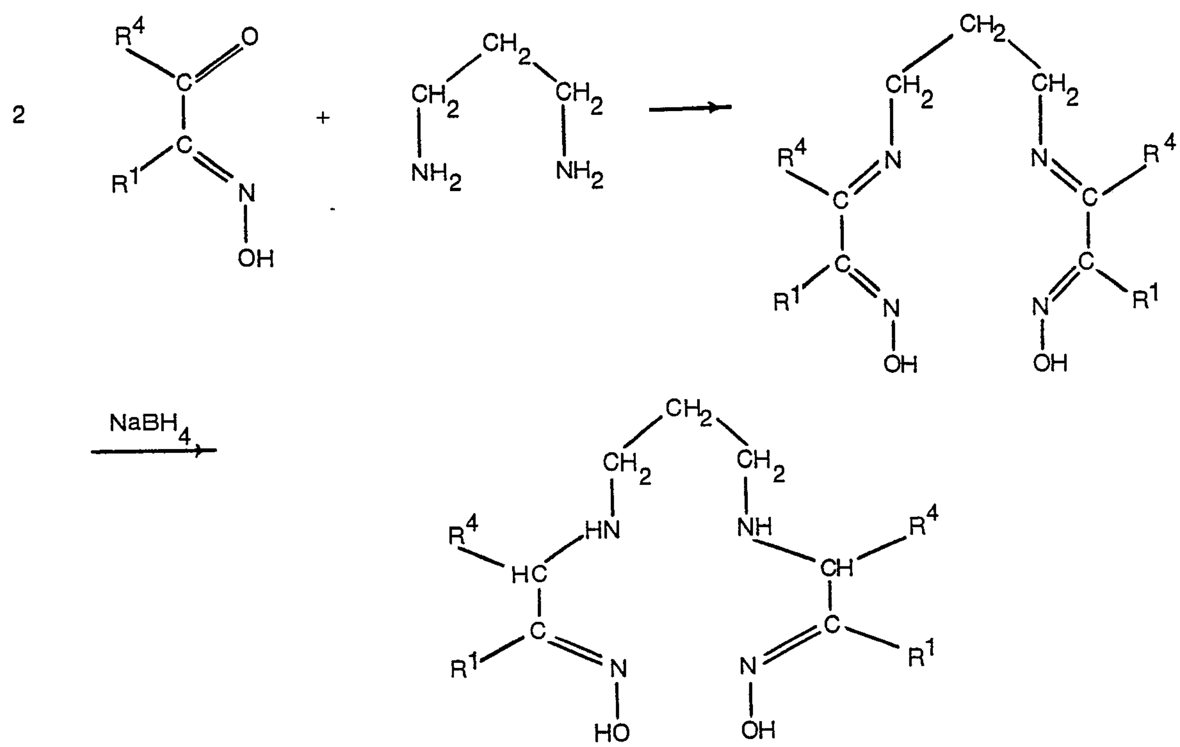

Propylene amine oxime or PnAO derivatives where substituent groups are introduced at the 2-carbon position of the propylene moiety can be prepared from diethylmalonate. A general scheme is outlined below for the synthesis by this route and involves the alkylation of the C-2 carbon followed by conversion of the diester functionalities to diamides with subsequent reduction to the 1,3-propane diamine derivative. Other routes from diester to diamine are possible, for example via the diol. The diamine product can then be reacted with the chloro-oxime reagent to form the respective PnAO derivative.

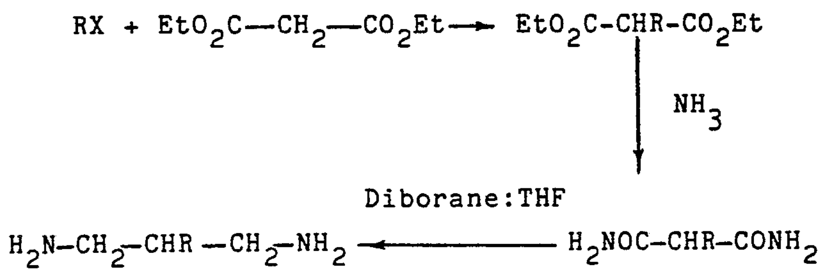


Alternatively, analogue derivatives can be made by a similar reaction where 2-OH-diethylmalonate is reacted with RX. In this case the R group is attached to the C-2 propylene carbon by an ether linkage instead of directly to the C-2 propylene:

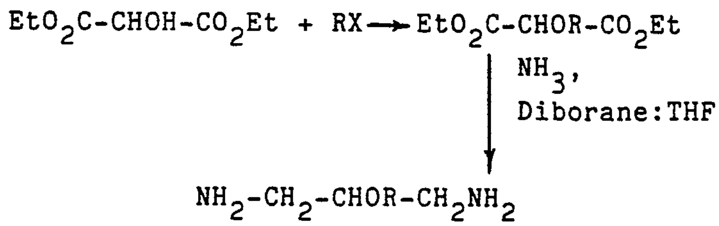


As before, the ester groups are converted to the amines and reacted with excess chloro-oxime to form the respective PnAO derivative.

An alternative way to form hydrophobic 1,3-propane diamine derivatives is to attach groups to the C1 and C3 carbon atoms of the propylene diamine. These derivatives can be made by reacting alpha-gamma-diketones (e.g., acetylacetone) with $NH_3$, followed by reduction to produce the following 1-3-propane diamine derivatives and subsequent reaction with the chlorooxime reagents.

The other principal starting material is the technetium-99m as pertechnetate ($TcO_4^-$-99m). The pertechnetate reagent is freshly generated as the sterile, pyrogen free eluate from a standard molybdenum-99 (Mo-99): Tc-99m radionuclide generator. In accordance with standard practice, the $TcO_4^-$-99m is eluted from the column using aqueous physiological saline, such as isotonic (0.9M) NaCl. The eluate will contain a dilute solution of the pertechnetate, such as a molar concentration of about $10^{-6}$—$10^{-9}$.

The complexation reaction between the propylene amine oxime ligand and the pertechnetate reagent may be carried out in an aqueous solution or a solvent mixture composed of isotonic saline and a water-miscible organic solvent under reducing conditions. Standard reducing agents can be employed, such as those that have been previously used in the reduction and ligand complexing of Tc-99m in commercial "kit-type" preparations. For example, suitable reducing reagents include; $SnCl_2$ and other stannous salts, sodium dithionite, sodium borohydride, formamidine sulphuric acid, or Sn or Zr electrodes, etc. Even though Tc-99m-PnAO can be prepared by a variety of reducing conditions (for example, we have prepared Tc-99m-PnAO by reducing $TcO_4^-$-99m in physiological saline at pH 8—10 in the presence of 0.004*M* PnAO with sodium dithionite), stannous salts are the most convenient method of preparation. The use of stannous salts is a particularly suitable means for providing the reducing agent in pre-formulated radiopharmaceutical "kits" for routine use in patients. In these "kits", the lyophilyzed contents of a sterile pyrogen free vial containing the stannous salt, complexing ligand and other chemical, possibly a buffer and stabilizing agent, are combined with the eluate from a Mo-99-Tc-99m generator containing $TcO_4^-$-99m. The resulting Tc-99m chelate can then be injected into the blood stream of a patient.

Since the complexes of this invention contain Tc-99m bound rather strongly, they can also be prepared by a process of ligand exchange. That is to say, the pertechnetate can be reduced initially in the presence of some different ligand that binds technetium relatively weakly. When the resulting Tc-99m-ligand complex is subsequently mixed with PnAO, the Tc-99m transfers to the PnAO and forms a complex according to this invention.

The complexation reaction of Tc-99m with PnAO is not highly sensitive to pH as long as either high acid or basic conditions are avoided. For example, the complexation reaction can be carried out at a pH of 5 to 10. In this pH range, complexation yields of greater than 95% are achieved by reducing $TcO_4^-$-99m with stannous ion with excess PnAO in saline solution. The ligand concentration in saline at the time of $TcO_4^-$-99m addition can be as low as $10^{-5}$*M*. The pH adjustment can be made with a variety of reagents, such as sodium bicarbonate, sodium acetate, sodium borate, or other salts suitable for buffering in this pH range.

The temperature of the reaction is not critical, and may be carried out at room temperature. However, the rate of the reaction may be promoted, if desired, by using a temperature as high as 90°C.

The desired complexing reaction occurs rapidly, and will usually be completed within 10 minutes. If desired, the completion of the reaction may be determined by a suitable test procedure, such as paper chromatography, electrophoresis, thin layer chromatography or high performance liquid chromatography (HPLC).

Brain Imaging Agents

In *in vivo* behaviour of these complexes in animals indicates their potential wide spread applicability for the formulation of new and useful radiopharmaceuticals. A bolus intravenous injection of Tc-99m PnAO in laboratory animals (i.e., mice, rats, rabbits, monkeys, and dogs) showed a high uptake by the brain and lung within a few seconds after injection. The activity in the brain decreases rapidly and becomes relatively low by 15 minutes. This uptake and washout of Tc-99m PnAO from the brains of dogs, rabbits, and rats was clearly visualized using a standard scintillation camera and interfaced directly to a computer. The images were digitized and processed by the computer. The activity expressed as a function of tims of the Tc-99m-PnAO in the brain was evaluated by plotting the activity outlined by the region of interest (ROI) of the brain compared to an ROI over non-brain tissue against time. The rate of Tc-99m PnAO washout in the dog was similar to that observed with clearance of Xe-133 from the brain in humans. This data indicates that the brain uptake of Tc-99m-PnAO is efficient and that measurement of washout rates of this chelate from various areas of the brain with the proper instrumentation is, indeed, feasible. Furthermore, the specific SPECT instrumentation (1), multiprobe systems (2) currently used to follow the rate of Xe-133 brain clearance in humans can also be applied to determine regional clearance of Tc-99m-PnAO from the brain and determine rCBF patterns in patients. Even though less satisfactory, diagnostic information by planar imaging of the Tc-99m-PnAO uptake in the brain can also be obtained.

The extraction efficiency of Tc-99m-PnAO from the plasma by the brain of rats and rabbits was determined by using a single probe external gamma-ray detection system. The extraction efficiency in these animals at presumed normal blood flow, was determined to be about 70—90%. The extraction efficiency was also measured in monkeys using an external probe system, and was found to be 80% at normal blood flow. As far as is known, this extraction efficiency is superior to any neutral or charged lipophilic Tc-99m complex known. The ability of this complex to readily diffuse across intact cellular membranes was confirmed by the observation that Tc-99m-PnAO placed into a whole-blood sample (Hct=45) will reach an equilibrium concentration of about 70% inside the red cells in less than 5 minutes. Based on these results, Tc-99m-PnAO and various derivatives or other tetradentate amine oximes (e.g., EnAO derivatives) maintain the greatest potential of any known Tc-99m-chelates that could be used as a radiopharmaceutical to assess patterns of regional cerebral blood flow (rCBF) in humans.

Tc-99m-PnAO (or Tc-99m-EnAO) could be used to assess rCBF using commercially available Nuclear Medicine instrumentation which has the capability of following the rate of washout of activity (e.g., Xe-133) from various regions of the brain. For example, SPECT images can be obtained using a specialized Tomographic Unit (Tomomatic Model 64 Medimatic Corp., Irvine, CA) while the multiprobe system (Novo Cerebrograph marketed by Novo Laboratories, Inc., Wilton, CT) can be used to follow washout patterns in localized brain areas.

An alternative type of clinically useful rCBF agent is one that will passively diffuse across the intact BBB from the plasma and be retained intracellularly. The intracellular retention should be enough to permit imaging by conventional SPECT instrumentation. The TC-99m-PnAO chelates proposed for this use are structural analogues of the I-123-iodophenyl alkylamine derivatives. Specifically, Compounds A and B have been proven to be useful rCBF imaging agents (I in each Structure = I-123).

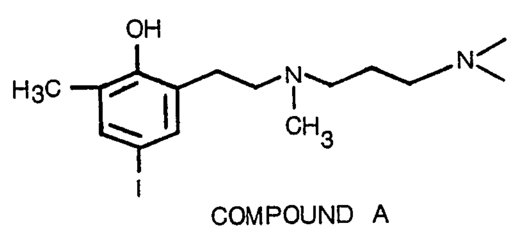

COMPOUND A

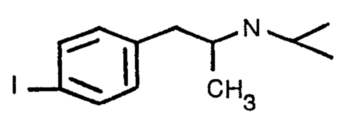

COMPOUND B

The Tc-99m-PnAO chelates that will be useful for this purpose will in essence be designed to replace the I-123-phenyl substituent with the neutral-lipophilic Tc-99m-PnAo (or a PnAO analogue) substituent using the same or similar side chains attached as shown for Compounds A & B. The most straight forward methods to attach substituents to PnAO (or one of its analogues) is to attach the chain to the C-2 propylene by the diethylmalomate synthetic route described earlier. The substituent, attached either directly to or via an ether linkage to the C-2 propylene will be an alkyl amine or polyamine chain that may contain from 4—10 carbons. If the polyamine and alkyl amine chains that are attached to the phenyl ring in Compound A & B, respectively, are attached to PnAO (or a suitable analogue), they should produce a very desirable rCBF imaging agent. However, other alkyl amine and poly amine groups attached to PnAO may be superior. For examples of such side chains see Winchell et al., (J. Nucl. Med., 20, 940—946, 1980).

Two examples of synthesis using the diethylamonate synthetic route are outlined as follows: The PnAO analogue with a structure similar to Compound A can be prepared by reacting 2-(cyanoethyl)diethylmalonate with ammonia and subsequent reduction with sodium borohydride to form 2-(ethylamine)malondiamide. This compound can then be reacted with $ClCH_2CH_2CH_2N(CH_3)_2.HCl$ and then reduced with diborane to form the 1,3-propanediamine derivative. One part of this propane diamine derivative is then reacted with two parts of a chloro-oxime reagent to produce the desired PnAO derivative.

The second example involves reaction of the preformed Br-alkyl amine salt (i.e., $BrCH_2CH(CH_3)NHCH(CH_3)_2.HBr$) with diethylmalonate followed by amidation, reduction with diborane and subsequent reaction of the 1,3-diamino propane derivative with a chloro-oxime reagent.

## Myocardial Imaging Agents

Fatty acid analogues labelled with I-123 have been shown to be effective for myocardial imaging in humans. Myocardial uptake and oxidative metabolism of these compounds is not critically dependent upon subtle structural differences. In fact, long chain fatty acid analogues that have bulky I-123-phenyl groups attached to the w-end by a variety of linking groups are taken up by heart muscle. For this reason, replacement of the I-123-phenyl substituent with Tc-99m-PnAO (or a Tc-99m PnAO analogue) should produce an agent with similar myocardial uptake because of the exceptional ease in which this neutral-hydrophobic chelate passes through biological membranes. Fatty acid analogues with PnAO attached to the w-end, can be made using the diethyl malonate synthetic route outlined earlier. Once the 1,3-diamino propane derivatives have been made, PnAO, or PnAO analogues can be produced by reacting them with one of the chlorooxime reagents. For example, Tc-99m-PnAO-hexadecanoic acid is an analogue of w-I-123-phenyl-hexadecanoic acid which is known to localize in myocardial tissue. This general type of compound is taken up rapidly by heart muscle and clears because of beta-oxidation. By following the rate of regional myocardial clearance, one can obtain a localized oxidative myocardial metabolic index. A variety of atoms or groups can be used to link the fatty acid analogue side chains to the tetradentate amine oxime chelating moiety. It has been shown that attaching the I-123-phenyl group to the omega-end of several fatty acid analogues with ether, amide, ester, thioether, amine, and sulfonimide linkages did not significantly alter their uptake in the myocardium of rats.

Several structural modifications have been made to trap I-123 labelled fatty acid analogues in heart muscle to permit planar and tomographic imaging over extended times. Similar modifications can be made for the w-PnAO fatty acid derivatives. For example, it is known that the addition of a methyl group to the beta-position on a w-I-131-phenyl fatty acid analogue (i.e., w-I-131-phenyl-b-methyl-tetradecanoic acid) produces a derivative that concentrates in normal myocardium and has a retention time sufficiently long to permit conventional SPECT imaging. A labelled fatty acid analogue, such as this, that is partially metabolized in the myocardium would be very useful for imaging regional metabolism in the heart. The addition of the small alkyl side chain in the beta-position inhibits beta-oxidation of fatty acids. Thus, an w-Tc-99m-PnAO derivative of a fatty acid with an alkyl substitution in the C-3 position should behave *in vivo* in a manner similar to the beta-methyl-w-I-123-phenyl fatty acid derivatives and find widespread applicability for metabolic imaging of the myocardium. Furthermore, one or two alkyl groups can be added to carbon atoms on the fatty acid chain other than at the beta-position which will cause metabolic trapping. To interrupt beta-oxidation, the alkyl groups must be on carbon atoms which are multiplets of 2 away from the beta-carbon.

It is essential to recognize that fatty acid analogues have limited solubility in aqueous solutions. Therefore, the feasibility of producing "kit" type formulations for routine human use demands that the Tc-99m-chelating group attached to the w-end of these analogues be able to form a stable chelate in high yields at low concentrations at or near neutral pH. The linear tetradentate amine oxime ligand (e.g., PnAO) is capable of forming complexes at >95% yields at $3 \times 10^{-5}M$ (See Example 19, Table 1). Because of this property, this type of ligand, as far as known, is the only one that will allow the production of a wide variety of Tc-99m-labelled fatty acid analogues with a stable-neutral-lipophilic chelate at the w-end by "kit"-type formulation.

## Lung Imaging Agents

Tc-99m labelled compounds that are extracted from plasma by lung tissue may be useful in assessing various lung diseases. Tc-99m-PnAO has demonstrated significant lung tissue localization.

Because Tc-99m-PnAO clears the blood and does not accumulate significantly in heart muscle, the

lung/blood and lung/heart ratios are both approximately 10/1 at 15 seconds post injection (these ratios are high enough to permit good lung imaging). All of the Tc-99m complexes of PnAO or its derivatives (PnAO analogues) that were outlined for use in brain images can also be used to assess lung disease. The alkyl derivatives (used to measure rCBF patterns by determining regional brain clearance) can be used to assess lung diseases where the uptake and washout of a neutral-lipophilic Tc-99m-Pharmaceutical is required (e.g. to determine differences in extravascular lung water). Tc-99m-PnAO, in its underivatized form should be suitable for these studies.

The alkyl amine and polyamine derivatives proposed for brain imaging which are retained long enough for imaging with conventional SPECT instrumentation (e.g., Tc-99m-chelate analogues of Compounds A and B) can also be used. These latter types of compounds would have lengthened lung retention (similar to Compound B because of their apparent binding to high-affinity amine binding sites located on the membranes of pulmonary endothelial cells. This type of Tc-99m compound could provide a method to routinely evaluate the metabolic function of the lung, especially the lungs effect on concentration of circulating bioamines. As previously stated, the tetradentate amine oxime ligand provides a unique opportunity to produce "kit"-type formulations of Tc-99m-labelled compounds for both brain and lung-imaging in clinical Nuclear Medicine.

EnAO will also form a neutral-lipophilic stable Tc-99m complex that passively diffuses across the intact BBB and other lipid-bilayer membranes. Accordingly, derivatized forms of EnAO with various substituents attached to the tetradentate ligand back bone, similar to the structures proposed for PnAO derivatization, can be synthesized. The only difference would be that substituents are attached to either one or both ethylene carbons instead of the propylene carbons in PnAO. These derivatives can be made by forming ethylene diamine derivatives as outlined below:

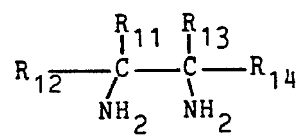

The ethylene diamine derivatives will react with a chloro-oxime reagent to form various substituted EnAO ligands. For example, $R_{11}$ can be a fatty acid analogue side chain of 6 to 22 carbons linked by various groups (e.g. amides, ethers, etc.) with $R_{12}$, $R_{13}$, and $R_{14}$ being H atoms or one or more small (1—3 carbons) alkyl groups. $R_{11}$ and $R_{13}$ can be small alkyl side chains (1—5 carbons) with $R_{12}$ and $R_{14}$ equal to hydrogen (H) and formed using alpha-beta diketones. Alternatively, $R_{11}$ can be an alkyl or polyamine chain (with only secondary or tertiary amines). Tc-99m complexes of EnAO derivatives should have similar *in vivo* behaviour to the analogues Tc-99m-PnAO derivatives.

PnAO does not appear to be toxic. Swiss-Webster mice injected intravenously with approximately 1000—1500 times the anticipated mg/kg dose for humans showed no measurable or visible acute effects (see Example 21). This implies that these types of ligands, their derivatives and their Tc-99m-chelates will be safe to use in diagnostic Nuclear Medicine Procedures.

The following Examples illustrate the invention. Examples 1 to 17 describe preparation of EnAO, PnAO and derivatives of PnAO having the structure

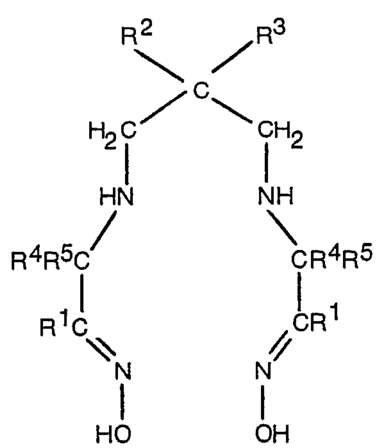

| Example | $R^1$ | $R^2$ | $R^3$ | $R^4$ | $R^5$ |
|---|---|---|---|---|---|
| 2 | $CH_3$ | H | H | $CH_3$ | $CH_3$ |
| 3 | $C_2H_5$ | H | H | $CH_3$ | $CH_3$ |
| 4 | $n-C_4H_9$ | H | H | $CH_3$ | $CH_3$ |
| 5 | $n-C_9H_{19}$ | H | H | $CH_3$ | $CH_3$ |
| 6 | $CH_3$ | $CH_3$ | $CH_3$ | $CH_3$ | $CH_3$ |
| 7 | $CH_3$ | OH | H | $CH_3$ | $CH_3$ |
| 8 | $CH_3$ | morpholine | H | $CH_3$ | $CH_3$ |
| 9 | $CH_3$ | piperadine | H | $CH_3$ | $CH_3$ |
| 10 | $CH_3$ | morpholinylethyl | H | $CH_3$ | $CH_3$ |
| 11 | $n-C_6H_{13}$ | H | H | $CH_3$ | $CH_3$ |
| 12 | $n-C_8H_{17}$ | H | H | $CH_3$ | $CH_3$ |
| 13 | $n-C_3H_7$ | H | H | $CH_3$ | $CH_3$ |
| 14 | $CH_3$ | $Et(Ph)NCH_2CH_2-$ | H | $CH_3$ | $CH_3$ |
| 15 | $CH_3$ | H | H | $CH_3$ | H |
| 16 | $CH_3$ | $C_5H_{11}$ | H | $CH_3$ | $CH_3$ |
| 17 | $CH_3$ | hexadecanoic acid | H | $CH_3$ | $CH_3$ |

## Example 1

### Preparation of 4,7-Diaza-3,3,8,8-tetramethyldecane-2,9-dione bisoxime (EnAO)

Synthesis of this ligand follows the method of R. K. Murmann (J. Amer. Chem., Soc., 1958, *80,* 4174).

a) 3-Chloro-3-methyl-2-nitrosobutane

2-Methyl-2-butene (18.5 ml, 175 mmol) and *iso*-amyl nitrite (19.5 ml, 131 mmol) were mixed in a three necked flask at −10°C. Concentrated hydrochloric acid (17.5 ml) was added dropwise to the stirred mixture, maintaining a temperature below 0°C. Stirring was continued for ½ hour after the addition was complete. The precipitated solid was filtered and washed well with chilled ethanol (4 × 5 ml). The solid was dried in a stream of air until all traces of the blue colouration had gone. Drying *in vacuo* gave the crude product as a white crystalline solid 8.63 g, 36%. Recrystallisation from methanol at −80°C gave a sharp melting solid (72.5—74°C).

b) EnAO

Ethylenediamine (485 microlitre, 7.25 mmol) was added slowly to a stirred, chilled slurry of 3-chloro-3-methyl-2-nitrosobutane (2.171 g, 16 mmol) in methanol (10 ml). The resultant mixture was stirred at ambient temperature overnight, then heated under reflux for 8 hours. The solvent was removed *in vacuo*, and the residue was dissolved in a minimum quantity of cold water. The solution was filtered, and treated with a slight excess quantity of solid sodium bicarbonate. A white crystalline solid was deposited after standing at 2°C for 72 hours. The solid was filtered, washed with ice water, and dried *in vacuo.*. Yield 272 mg (70.3%), m.pt. 180—1°C.

## Example 2

### Preparation of 4,8-Diaza-3,3,9,9-tetramethylundecane-2,10-dione bisoxime (PnAO)

This compound was prepared according to the procedure of E. G. Vassian and R. K. Murmann, Inorg. Chem., *6,* 2043 (1967).

3-chloro-3-methyl-2-nitrosobutane (3.8 g, 28 mmol) was slurried in dry methanol (10 ml) and cooled to 0°C, 1,3-Propanediamine (1.05 ml, 12.5 mmol) was added dropwise to the stirred suspension. The mixture was allowed to reach room temperature whilst stirring and then refluxed for 16 hours. The methanol was removed *in vacuo* and the solid residue slurried in a small quantity of water. The precipitate was filtered, washed with a little water and dried *in vacuo* giving the product as a white solid, 2.2 g, 58%. Recrystallisation from the minimum quantity of hot methanol gave a pure sample, 1.68 g, 44%, m.pt. 177—9°C.

Example 3

Preparation of 5,9-Diaza-4,4,10,10-tetramethyltradecane-3,11-dione bisoxime

This compound was prepared from 1,3-propane diamine and 2-chloro-2-methyl-3-nitrosopentane by the method described in Example 2. Yield 3.43 g (46%).

A sample was recrystallised from ethyl acetate/petrol m.pt. 140—1°C.

$^1$H-NMR (200 MHz, DMSO-$d_6$, δ ppm): 1.0 (t, 6H), 1.5 (s, 12H), 2.15—2.36 (m, 8H), 3.2 (6s, 2H), 10.35 (s, 2H).

$^{13}$C NMR (DMSO-$d_6$): 10.9, 17.2, 25.8, 31.4, 41.2, 57.0 and 163.8 ppm.

IR (KBr dis): 3300, 3190, 3050, 2990, 2060, 2930, 2870, 1650, 1460, 1390, 1380, 1365, 1180, 1140, 1070, 1045, 1000, 950, 830, 780 and 650 $cm^{-1}$.

(In this and following examples, NMR data are in ppm and IR data in $cm^{-1}$).

Example 4

Preparation of 7,11-Diaza-6,6,12,12-tetramethylheptadecane-5,13-dione bisoxime

The compound was prepared by reaction of 1,3-propanediamine and 2-chloro-2-methyl-3-nitrosoheptane by a method similar to that described in Example 2. Yield 60% m.pt. 170—172°C (dihydrochloride salt), $^1$H NMR (free base, 200 MHz, $CDCl_3$, δ ppm): 0.96 (t, 3H), 1.26 (s, 12H), 1.20—1.70 (m, 12H), 2.27 (m, 4H), 2.45 (t, 2H).

Example 5

Preparation of 12,16-Diaza-11,11,17,17-tetramethylheptacosane-10,18-dione bisoxime

This compound was synthesized from 1,3-propanediamine and 2-chloro-2-methyl-3-nitrosododecane by the method described in Example 2. Yield 37% m.pt. 185—186°C (dihydrochloride salt), $^1$H NMR (free base, 60 MHz, $CDCl_3$, δ ppm): 2.55 (4H), 2.25 (m, 4H), 1.0—2.0 (m, 44H), 0.9 (dt, 6H).

Example 6

Preparation of 4,8-Diaza-3,3,6,6,9,9-hexamethylundecane-2,10-dione bisoxime

This ligand was prepared in 41% yield by the reaction of 2,2-dimethyl-1,3-propanediamine and 2-chloro-2-methyl-3-nitrosobutane using the method described in Example 2. m.pt. 151—2°C.

Example 7

Prepartion of 4,8-Diaza-6-hydroxy-3,3,9,9-tetramethyl-2,10-dione bisoxime

The compound was prepared from 2-hydroxy-1,3-propanediamine and 2-chloro-2-methyl-3-nitrosobutane by the method described in Example 2. Yield 37%, m.pt. 139—40°C.

$^1$H NMR (60 MHz, DMSO-$d_6$ δ ppm): 3.7 (m, 1H), 2.35 (m, 4H); 1.8 (s, 6H), 1.2 (s, 12H).

Example 8

Preparation of 4,8-Diaza-6-N-morpholinyl-3,3,9,9-tetramethylundecane-2,10-dione bisoxime

a) Diethyl morpholinylmalonate

To a stirred solution of morpholine (19.7 g, 0.22 mol) in dry acetonitrile at room temperature was added diethyl bromomalonate (23.90 g, 0.1 mol). When the addition was complete, the reaction mixture was refluxed for 2 hours. After cooling the precipitated salt was filtered off and washed with dichloromethane. The filtrate was concentrated, water and dichloromethane were added and the pH of the solution was adjusted to pH 10. The organic layer was washed with water, dried and concentrated. The resulting orange oil was distilled to give the desired compound as a clear oil (18.40 g, 75%). Bp 99—104°C at 0.04—0.06 mmHg.

$^1$H NMR (60 MHZ, $CDCl_3$.δ ppm): 1.0—1.7 (t, 6H), 2.6—2.98 (m, 4H), 3.6—3.9 (m, 4H), 4.0 (s, 1H) and 4.05—4.4 (q, 4H).

IR (film): 2980, 2880, 1750, 1445, 1300, 1265, 1225, 1160, 1120, 1040 and 860 $cm^{-1}$.

b) Morpholinylmalonamide

To a stirred solution of diethylmorpholinylmalonate (24.50 g, 0.1 mol) in dry methanol (250 ml) saturated with dry ammonia was added a catalytic amount of sodium (50 mg). After stirring overnight the solution was re-saturated with ammonia and was left standing for 3 days. The resulting white crystals were filtered off and washed with methanol and ether and dried *in vacuo.*

Yield 17.40 g (93%) m.p. 231—5°C. A sample was recrystallised from methanol m.pt. 234—8°C.

$^1$H NMR (360 MHz, DMSO-$d_6$, δ ppm): 2.58 (m, 4H), 3.65 (m, 5H), and 7.4 (m, 4H).

I.R. (KBr disc): 3400—3180, 1710, 1680, 1610, 1375, 1285, 1270, 1150, 1110 and 885 $cm^{-1}$.

c) 2-Morpholinyl-1,3-propanediamine

Boron trifluoride etherate (98 ml, 0.8 ml) in tetrahydrofuran (THF, 150 ml) was added dropwise to a cooled (0°C), stirred suspension of finely ground sodium borohydride (22.7 g, 0.5 mol) in THF (200 ml). After stirring under nitrogen at room temperature for 2 hours finely ground morpholinylmalonamide (31.6 g, 0.17 mol) was added in small portions. The reaction mixture was stirred overnight and was heated under reflux for 8 hours. The reaction mixture was quenched by the cautious addition of a mixture of THF and 1M

hydrochloric acid (50 ml of 1:1 mixture) and 1M hydrochloric acid (10 ml). After stirring for 1½ hours the reaction mixture was evaporated to dryness and the boron-nitrogen complex was cleaved by refluxing with 5M hydrochloric acid (250 ml) for 6 hours. Neutralisation with sodium hydroxide pellets and saturation of the aqueous phase with salt and sodium hydroxide gave a white precipitate. This was filtered off and thoroughly washed with ether and the saturated aqueous phase was extracted with ether (10 × 30 ml). The organic layers were combined, dried ($Na_2SO_4$) and concentrated to give an orange oil (4.73 g). The oil was purified by short-path distillation (oven temperature 120—150°C at 0.03—0.05 mmHg) to give a clear oil (2.75 g, 10%).

$^1$H NMR (60 MHz, MeOH-$d_4$, δ ppm): 2.4—2.8 (m, 9H) and 3.5—38 (m, 4H).

I.R. (film): 3460, 2950, 2850, 1600, 1450, 1290 and 1115 $cm^{-1}$.

d) 4,8-Diaza-6-N-morpholinyl-3,3,9,9-tetramethylundecane-2,10-dione bisoxime

Preparation of the ligand was achieved by reaction of 2-morpholinyl-1,3-propanediamine and 2-chloro-2-methyl-3-nitrosobutane by the method described in Example 2. Yield 19%, m.pt. 167—9°C.

$^1$H NMR (200 MHz, DMSO-$d_6$, δ ppm): 1.12 (s, 12H), 1.68 (s, 6H), 2.1—2.5 (m, 9H), 3.33 (s, 4H), 3.53 (s, 2H) and 10.39 (s, 2H).

I.R. (KBr disc): 3420, 2970, 2930, 2850, 1450, 1380, 1370, 1140, 1125, 1010 and 935 $cm^{-1}$.

Example 9

4,8-Diaza-6-N-piperadinyl-3,3,9,9-tetramethylundecane-2,10-dione bisoxime

a) Diethyl piperidinylmalonate

Prepared similarly to ethyl morpholinylmalonate to give a clear oil in 83% yield. bp 84—88°C at 0.06—0.07 mmHg.

$^1$H NMR (60 MH, $CDCl_3$, δ ppm): 1.1—1.3 (t, 6H), 1.3—2.0 (m, 6H), 2.6—3.0 (m, 4H), 4.1 (s, 1H), and 4.15—4.5 (q, 4H).

I.R. (film): 2990, 2940, 1750, 1300, 1240, 1170, 1125, and 1035 $mm^{-1}$.

Piperidinylmalonamide

Prepared in a similar fashion to morpholinyl malonamide to give whole crystals. Yield 90%. m.pt. 259—60°C (from MeOH).

$^1$H NMR (360 MHz, DMSO-$d_6$, δ ppm): 1.45 (bs, 4H), 3.3 (s, 1H), and 7.25 (d, 4H).

I.R. (KBr disc): 3395, 3195, 1670, 1375, 1140 and 690 $cm^{-1}$.

c) 2-piperidinyl-1,3-propanediamine

Prepared in a similar fashion to 2-morpholinyl-1,3-propanediamine.

Short-path distillation (oven temp. 80°C at 0.06 mmHg) gave a clear oil yield 6%.

$^1$H NMR (200 MHz, MeOH-$d_4$ δ ppm): 1.3—1.6 (m, 6H) and 2.3—2.8 (m, 9H).

I.R. (film): 3360, 3280, 2915, 2850, 2800, 1600, 1460, 1455, 1445, 1150 and 1100 $cm^{-1}$.

d) 4,8-Diaza-6-(N-piperadinyl)-3,3,9,9-tetramethylundecane-2,10-dione bisoxime

Prepared in a similar fashion to the 5-morpholinyl derivative. The crude material crystallised from the final basic aqueous/dichlormethane mixture and was recrystallized from MeOH/$H_2O$. Yield 16%. m.pt. 168—9°C.

$^1$H NMR (250 MHz, MeOH-$d_4$, δ ppm): 1.1 (d, 12H), 1.4 (b.s. 6H), 1.7 (s, 6H) and 2.0—2.5 (m, 9H).

I.R. (KBr disc): 3400, 2930, 2850, 1330, 1140, 1020 and 935 $cm^{-1}$.

Example 10

Preparation of 4,8-Diaza-6-(N-morpholinylethyl)-3,3,9,9-tetramethylundecane-2,10-dione bisoxime

a) Morpholinoethyl-diethyl malonate

Sodium (5.0 g, 217 mmol) was weighed into a dry 3-neck flask fitted with a condenser and dropping funnel. Dry ethanol (125 ml) was added to dissolve the sodium. Diethylmalonate (33.0 ml, 217 mmol) was added and the mixture refluxed for 2 hours. Morpholinoethyl chloride (32.6 g, 217 mol) was added and the mxiture refluxed for 4½ hours.

After cooling to room temperature all solvents were removed *in vacuo* and the residue partitioned between dilute HCl (aq) and ether. The acid layer was washed with ether then adjusted to pH 14 with sodium hydroxide pellets. The aqueous layer was extracted with ether (3 × 50 ml). The combined ether extracts were dried ($MgSO_4$) and concentrated *in vacuo*. Vacuum distillation of the residue gave the product as a colourless oil, 6.6 g, 11%, bp 110°C/0.15 mmHg.

b) 2-Morpholinoethyl malonamide

The above diester (14.3 g, 53 mmol) was dissolved in methanol (35 ml) and saturated with ammonia gas. Sodium metal (5 mg) was added and the flask stoppered and set aside.

After 3—4 days the precipitate was filtered, washed with ether (3 × 10 $cm^3$) and dried *in vacuo* giving the product as a white crystalline solid, 7.3 g, 64%.

c) 2-Morpholinoethyl-1,3-Propanediamine

2-Morpholinoethyl-malonamide (899 mg, 4.2 mmol) was slurried in dry THF (20 ml) and thoroughly degassed under argon. Borane-tetrahydrofuran complex (27 ml of 1M solution) was added dropwise over ½ hour to the stirred, cooled (0°C) mixture. The mixture was allowed to reach room temperature and then refluxed for 3½ hours. The solution was cooled to 0°C and 5M HCl (aq, 6 ml) added dropwise by syringe. After standing at room temperature overnight the THF was removed *in vacuo*. The filtered aqueous solution was saturated with NaOH and extracted with ether (3 × 50 ml). The combined ether extracts were dried ($MgSO_4$) and concentrated *in vacuo*. Short path vacuum distillation of the residue gave the product as a clear liquid, 353 mg 45%; bp 75°C/0.2 mmHg.

d) 4,8-Diaza-6-(N-morpholinylethyl)-3,3,9,9-tetramethylundecane-2,10-dione bisoxime

The ligand was prepared by reaction of 2-morpholinylethyl-1,3-propanediamine and 2-chloro-2-methyl-3-nitrosobutane as described in Example 2. m.pt. 103—5°C.

The ligands of Examples 11—14 were synthesised by the procedures outlined in Examples 2—10.

Example 11

9,13-Diaza-8,8,14,14-tetramethylhenicosane-7,15-dione bisoxime

Prepared as the dihydrochloride, m.pt. 197—198°C.

Example 12

11,15-Diaza-10,10,16,16-tetramethylpentacosane-9,17-dione bisoxime

Prepared as the dihydrochloride m.pt. 198°C (decomposes).

Example 13

6,10-Diaza-5,5,11,11-tetramethylpentadecane-4,12-dione bisoxime

Prepared as free base m.pt. 89—91°C.

Example 14

4,8-Diaza-6-(N-ethyl-N-phenylaminethyl)-3,3,9,9-tetramethylundecane-2,10-dione bisoxime

Prepared as the free base m.pt. 165—170°C (decomposes).

Example 15

4,8-Diaza-3,9-dimethylundecane-2,10-dione bisoxime

a) 2-Butanone 3,3'-(1,3-propanediyldinitriolo) bisoxime

2,3-Butandione monoxime (4.14 g, 41 mmol) was dissolved in ethanol (11 ml) at 70°C. 1,3-Propanediamine (1.7 ml, 20 mmol) was added dropwise by syringe and the solution stirred for 5 minutes. The solution was stirred for 4 hours whilst allowing to cool to room temperature. Cooling to 0°C induced crystallisation. The white solid was filtered, washed well with ether (3 × 10 ml) and dried *in vacuo* giving the product as a white solid, 2.50 g, 51% m.pt 89—90°C. A further recrystallisation from ethanol gave material of greater purity, m.pt. 91—2°C.

b) 4,8-Diaza-3,9-dimethylundecane-2,10-dione bisoxime

2-Butanone 3,3'-(1,3-propanediyldinitriolo) bisoxime (2.5 g, 12.1 mmol) was slurried in 95% aqueous ethanol (28 ml) at 0°C. Sodium borohydride (0.93 g, 25 mmol) was added in portions over ½ an hour and stirring continued for 1½ hours after the addition was complete. Water (8 ml) was added and the mixture stirred for ½ an hour. The ethanol was removed *in vacuo* and the aqueous residue diluted with more water. After adjusting the pH to about 10.5 using dilute hydrochloric acid the solution was extracted with $CH_2Cl_2$ (7 × 30 ml). The combined organic extracts were dried ($Mg SO_4$) and concentrated *in vacuo* giving the product as a clear oil (930 mg), which crystallised on standing. The solid was slurried in warm $CH_2Cl_2$ (4 ml), filtered, washed with ether (3 × 5 ml) and dried *in vacuo*. The product was thus obtained as a white crystalline solid, 450 mg, 18% m.pt. 119—122°C.

$^1H$ NMR (200 MHz, DMSO-$d_6$, δ ppm) 10.2 (2H, s, O—H), 3.2 (2H, q, CH), 2.3 (4H, t, $CH_2N$), 1.65 (6H, s, N=CMe), 1.44 (2H, m, $CH_2$), 1.04 (6H, d, $CH_3$).

Example 16

Preparation of 4,8-Diaza-6-pentyl-3,3,9,9-tetramethylundecane-2,10-dione bisoxime

a) 2-Pentyl diethylmalonate (I)

One gram sodium was dissolved in 10 ml of freshly distilled absolute ethanol. To this solution was added 7 ml of freshly distilled diethylmalonate. This suspension was heated to 70°C for two hours. To the collected suspension was added 5.5 ml of freshly-distilled bromopentane. The resulting mixture was slowly heated to reflux overnight. Ethanol was distilled from the mixture and the product collected by vacuum distillation (78°C—86°C at 4.5 mm Hg). Yield 4.5 ml.

Mass spectrum, m/e = 230 (M+).

NMR (60, MHz, $CDCl_3$), 0.8 (t, 3H), 1.1 (t, 12H), 1.7 (broad signal, 2H), 3.1 (t, 1H), 4.1 (9, 4H).

b) 2-Pentylmalondiamide (II)

Five grams of the above ester (I) was dissolved in methanol (25 ml) and a solution of ammonia in methanol (50 ml of a solution saturated at 0°C) containing sodium methylate (from 50 mg sodium). The mixture was stirred at room temperature for 90 hours. A white precipitate was collected after exhaustive washing with hot methanol.

Yield 70%. m.pt. 185°C. Mass spectrum, m/e = 172 (M+).

I.R. 3450, 3374 (N—H); 1690 (C=O), 1600 (N—H).

NMR (60 MHz, DMSO-$d_6$), 0.8 (t, 3H), 1.1 (m, 6H), 1.6 (br. signal, 2H), 7 (s, br, 2H), 7.2 (s, br, 2H).

c) 2-Pentylpropanediamine (IV)

Two grams of (II) was suspended in 40 ml freshly distilled THF. The suspension was cooled to 0°C and 40 ml of diborane:THF complex was gradually added. The suspension was stirred at room temperature for about 20 hours and the temperature then raised to reflux. After one hour of reflux, the resulting suspension was cooled to room temperature and 10 ml of 5N HCl was added. The THF was removed by distillation. The resulting thick residue was saturated with sodium hydroxide pellets and the suspension extracted with petroleum ether. The ether extract was washed with water and dried over sodium sulfate. The product was examined by TLC using a plate developed with methanol/ammonium hydroxide (100:1) and stained with copper sulfate. A single blue spot developed upon mild heating.

I.R. 3400, 3300 (N—H), 1670, 1600 (N—H).

NMR (60 MHz, $CDCl_3$), 0.8 (t, br, 3H), 1.1 (s, br, 8H), 1.6 (br, 1H), 2.5 (br, 4H), 3.5 (br, 4H).

d) 4,8-Diaza-6-pentyl-3,3,9,9-tetramethylundecane-2,10-dione bisoxime (V)

140 mg of (IV) was dissolved in 5 ml of freshly distilled methanol and 290 mg of 3-chloro-3-methyl-2-nitrosobutane was added. The resulting mixture was held at 0°C for 2 hours and then brought to room temperature. After 2 hours at room temperature the mixture was refluxed overnight. The resulting solution was concentrated under reduced pressure and suspended in chloroform. This suspension was filtered and the filtrate examined by TLC as above. One green spot with $R_f$ greater than that of (IV) was developed.

IR 3250 (O—H), 1675 (C=N), 1600 (N—H).

NMR (60 MHz, $CDCl_3$), 0.8 (t), 1.1 (s, br), 1.3 (s, br), 1.8 (s, br), 2.3 (m, br), 3.0 (br), 3.5 (m, br), 9 (d, br).

Example 17

Preparation of 4,8-Diaza-6-(15-carboxy-n-pentadecyl)-3,3,9,9-tetramethylundecane-2,10-dione bisoxime

a) 16-bromohexadecanoic acid

Ten grams of 16-hydroxydecanoic acid was dissolved in 100 ml of 30% hydrobromic acid in glacial acetate acid and refluxed for 6 hours. The mixture was dried under reduced pressure and finally vacuum distilled at 100°C (4.8 mm Hg). The residue of the distillation product was crystallized from petrol ether. Yield 90%. m.p. 69—69.5°C.

I.R. (film) 1675 (C=O).

NMR (60 MHz, $CDCl_3$), 1.5 (s, br, $CH_2$), 2.3 (m, $CH_2$—C=O), 3.4 (t, $CH_2$—Br), 9 (br, COOH).

b) 2-(15-carboxy-n-pentadecyl)-diethyl malonate (VII)

One gram of sodium metal was dissolved in 15 ml of freshly distilled absolute ethanol. Seven grams of diethyl malonate was then added to the cooled solution of sodium ethoxide. The resulting suspension was refluxed for one hour and cooled to room temperature. Two grams of 16-bromohexadecanoic acid was added and the suspension refluxed overnight. The suspension was cooled and filtered after evaporation of most of the ethanol. The resulting white precipitate was exhaustively washed with cold ethanol.

I.R. (neat), 1750 (—C=O), ester), 1710 (—C=O, acid).

NMR (60 MHz, $CdCl_3$), 1.2 to 1.5 (s, br, $CH_2$), 2.3 (m, $CH_2$—C=O), 3.2 (m, O=C—CH—C=O), 3.4 (m, $CH_2$—Br), 4.1 (g, —$CH_2$—O—C=O).

c) Ammonium 17,17-bis(aminocarbonyl)heptadecanoate (VIII)

2.65 grams of 2-(hexadecanoic acid)diethyl malonate was suspended in 100 ml of freshly distilled methanol saturated with ammonia at 0°C. To this suspension was added 200 mg sodium in a solution of methanol and the resulting mixture stirred at room temperature for eighty hours. The product was recovered from boiling methanol by filtration. m.p. 225°C—230°C.

I.R. (nujol mull), 3400, 3200 (NH), 1700 (O=C—N), 1575 (O=C—O—).

NMR (60 MHz, DMSO-$d_6$), 1.2 to 1.5 ($CH_2$), 2.3 (m, —$CH_2$—C=O), 6.8 and 7.2 (s, br, $H_2N$—C=O). This doublet disappeared upon addition of $D_2O$.

Upon acidification of the above ammonium salt, the IR absorption at 1575 reappeared at 1675 and the m.p. changed to 184°C—186°C.

d) Ammonium 18-amino-17-aminomethyloctadecanoate (IX)

500 mg of VIII was suspended in distilled THF and stirred for 1 hour under reflux. The suspension was cooled and 8 ml of 1M diborane:THF complex was gradually injected. The milky suspension was stirred for 1 hour and then refluxed. All operations were carried out under nitrogen. After 3 hours, the reaction

mixture was cooled and ice was added. The resulting suspension was acidified with 5N HCl and the THF distilled off. The pH was adjusted to 12 ammonium hydroxide and the suspension filtered while hot. A white precipitate was collected after several washings with cold water. m.pt. 117°C—120°C.

I.R. (Nujol mull), 3400, 3200 (NH), 1675 (O=C—O—), 1600 (NH).

NMR (60 MHz, DMSO-$d_6$), similar to that for III except for loss of doublet at 6.8, 7.2).

e) 4,8-Diaza-6-(15-carboxy-n-pentadecyl)-3,3,9,9-tetramethylundecane-2,10-dione bisoxime will be completed by adding 2-chloro-2-methyl-3-butanone oxime to IX as in Example 16.

Examples 18 to 21 describe preparations and chemical properties of technetium complexes of EnAO, PnAO and PnAO derivatives.

## Example 18

### Preparation of Tc-PnAO solutions from Tc-99m

Two mg of PnAO was dissolved in 0.1 ml of $10^{-3}$ to $10^{-4}$M HCl (i.e., pH 3—4) and then brought to a pH of 8 by dropwise addition of 0.1M NaOH. To this solution was added 0.7 ml of Tc-99m (7 to 30 mCi as $NaTcO_4$) followed by 0.1 ml of $SnC_4H_4O_6$ ($10^{-4}$M) (stannous tartrate).

This resulted in a ligand concentration of $3.5 \times 10^{-4}$M. The solution was shaken and allowed to stand for 10 to 30 minutes. In some experiments dilution with saline or with acetate or bicarbonate buffers was used to study the effects of changes in ligand concentration or pH. Tc-99 ($5 \times 10^{-7}$M) or $5 \times 10^{-6}$M $NH_4TcO_4$) was also used to prepare Tc-99-PnAO.

As an alternate method, 2 mg of PnAO was slurried with 0.9 ml Tc-99m and 0.1 ml $SnC_4H_4O_6$ and held for 20 minutes at 80°C. The mixture was cooled and filtered.

### Preparation of Solid Tc-PnAO from Tc-99

A solution prepared or 10 ml $10^{-2}$M PnAO in $10^{-3}$ HCl, 1 ml of $2.8 \times 10^{-2}$M $NH_4TcO_4$(Tc-99), 1 ml generator-produced $NaTcO_4$ (1 mCi Tc-99m) as a tracer, and 2 ml 1M $NaHCO_3$ was mixed and heated at 80°C. Three 0.2-ml portions of $10^{-1}$M $SnCl_2$ were added at 20 minute intervals. A solid and an orange-brown solution resulted.

The solid was extracted with $CH_3OH$. Two drops of water were added to two ml of this extract and allowed to evaporate slowly. After four days, orange-red crystals appeared and one was selected for an x-ray diffraction structural study.

## Example 19

The complexes of Example 18 were subjected to test procedures which included high performance liquid chromatography (HPLC), ascending solvent paper chromatograph (PC), electrophoresis and solvent extraction. Crystal structure of solid Tc-PnAO was determined by x-ray diffraction.

### HPLC Separations

HPLC separations were done on a Hamilton PRP-1 column with a Beckman Series 332 dual pump gradient chromatograph system. The radioactivity in the eluent was detected by a NaI scintillation detector connected to a ratemeter. The signal from the ratemeter was directed to a Houston Instrument Co. Omniscribe B-5000 strip chart recorder and to a Hewlett-Packard Model 3390A integrator.

The liquid phase at the beginning of separation was 0.02M $NaH_2PO_4$ containing 2% $CH_3OH$. One-half minute after the injection of a 10-microlitre sample, THF was added at a gradient such that its volume % was 25 at 3.5 minutes. Elution was continued at the concentration until 8.0 minutes, at which time the liquid phase was restored to 2% $CH_3OH$ to equilibrate the column for the next separation. Flow rate was 2 ml/min for all phases of the separation. Reduced, hydrolysed Tc eluted at 0.7 minutes, $TcO_4^-$ at 1.1 minute, and the complex at 6.5 minutes. Percentage complex yields were determined from the areas under the peaks as measured by the integrator. The hydrophobicity of the complex is demonstrated by the necessity of using an organic solvent to elute it.

### Paper Chromatography and Electrophoresis

An alternate method of analysis employed the results of paper chromatography and electrophoresis.

Paper strips ($5 \times 120$ mm cut from Gelman saturation pads) were spotted with 5 microlitres of sample 1 cm from the bottom of the strip and developed with acetone and with saline. The solvent front was allowed to ascend to the top of the strip. The strip was cut into six 2-cm sections and the Tc-99m in each determined by counting in a NaI well counter. Electrophoresis was carried out with a Gelman Deluxe Power Supply and Chamber using 0.1M $NaHCO_3$ buffer at pH 8.5 and Beckman #320046 electrophoresis strips at 300 volts for 45 or 60 minutes. The strips were scanned with a Technical Associates scanner or cut into sections as above.

The percentage of pertechnetate and reduced Tc-99m can be determined from the activity in the electrophoresis anode peak and the saline paper chromatography origin section, respectively. A large electrophoresis peak at the origin in the absence of reduced Tc is evidence for a neutral complex.

Octanol/saline and $CHCl_3$/saline separation

Fifty microliters of a Tc-99m-PnAO complex prepared by one of the methods above was added to 5 ml of normal saline and the solution thoroughly mixed. One ml of this solution was added to 1 ml of n-octanol and vortexed for 1 minute and then centrifuged for 5 minutes. Ten-microlitre aliquots were withdrawn from each layer and counted in a NaI well scintillation counter. An 0.8-ml aliquot of the octanol layer was withdrawn and added to an equal volume of normal saline and the extraction and counting repeated as above. The results of this back extraction were used to compute the octanol/saline ratio. Similar experiments were done with $CHCl_3$/saline as an approximate measure of complex yield. A high extractions ratio into these solvents indicates hydrophobity of the complex.

Results

The effect of concentrations of pertechnetate PnAO, and $SnC_4H_4O_6$ on the complex yield is shown in Table 1.

Table 1

Effect of concentrations in complex yield

| Pertechnetate | (PnAO) | ($SnC_4H_4O_6$) | Yield (%) |
|---|---|---|---|
| Tc-99m | $3 \times 10^{-3}$ | $1 \times 10^{-5}$ | 98 |
| Tc-99m | $3 \times 10^{-4}$ | $1 \times 10^{-5}$ | 99 |
| Tc-99m | $3 \times 10^{-5}$ | $1 \times 10^{-5}$ | 95 |
| Tc-99m | $3 \times 10^{-6}$ | $1 \times 10^{-5}$ | 93 |
| Tc-99m | $5 \times 10^{-5}$ | $1 \times 10^{-6}$ | 98 |
| *$5 \times 10^{-7}$ | $5 \times 10^{-5}$ | $5 \times 10^{-6}$ | 97 |
| *$5 \times 10^{-6}$ | a | $2 \times 10^{-5}$ | 98 |

Tc-99m concentrations were in the range of $(0.4\text{-}2) \times 10^{-7}$M.

*Tc-99 was used for these studies.

Complex yields were measured 30 minutes after preparation using HPLC.

a) Saturated solution of PnAO at 50°C.

This demonstrates that the complex can be formed in high yield at pertechnetate concentrations equivalent to those normally encountered from commercial Mo-99-Tc-99m generators and low concentrations of PnAO and reducing agent.

The effect of pH on complex formations is shown in Table 2. Yields were measured 30 minutes after complex formation.

Table 2

Complex Yield as a Function of pH

| pH | Yield (%) | pH | Yield (%) |
|---|---|---|---|
| 3.2 | 76 | 8.5 | 98 |
| 4.0 | 85 | 10.0 | 98 |
| 5.5 | 92 | 11.0 | 91 |
| 7.0 | 98 | 12,0 | 80 |

The effect of pH on the stability of the complex stored in air is shown in Table 3. Complexes were prepared at pH 8.5 and then adjusted to the pH's shown. All were at least 98% complexed at the time of formation.

Table 3

Effect of pH on Complex Stability

| pH | Complex Yield (%) 1 hr. | 2 hr. | 24 hr. |
|---|---|---|---|
| 3.2 | 96 | 81 | 59 |
| 4.0 | 92 | 80 | 53 |
| 5.5 | 98 | 97 | 98 |
| 7.0 | 99 | -- | -- |
| 8.5 | 100 | 98 | 95 |
| 10.0 | 99 | 97 | 95 |
| 11.0 | 71 | 34 | 0 |
| 12.0 | 14 | 0 | 0 |

The complex can be formed in high yield over the pH range 5.5 to 10 and is stable up to 24 hours at those same pH's. It was also found that complexes prepared at pH 8.5 and diluted by a factor of 1000 with normal saline were also stable for 24 hours.

Validation that the complex is neutral and formed in high yields is demonstrated by the results in Table 4.

Table 4

Paper Chromatography and Electrophoresis of Tc-PnAO, $TcO_4$ and reduced Hydrolysed Tc. (Tc = both Tc-99 and Tc-99m).

| | Tc-PnAO | $TcO_4^-$ | Red. Tc |
|---|---|---|---|
| % Activity at origin in paper chromatography | | | |
| Acetone | ~2 | <1 | ~100 |
| Saline | <1 | <1 | ~100 |
| Migration distance in electrophoresis, cm | | | |
| Anode | 0 | ~8 | 0 |
| Cathode | 0 | - | 0 |
| % Activity at origin in electrophoresis | >97 | <1 | 100 |

There is a large peak at the electrophoresis origin, which is not reduced Tc-99m (shown by paper chromatograph), and is therefore the complex which must be neutral. Further proof of the hydrophobicity

is that the octanol/saline extraction ratio for Tc-PnAO (average of 5 trials) is 11 ± 33 and the $CHCl_3$/saline extraction ratio is 29 ± 5.

Tc-EnAO complex with Tc-99m

A complex of EnAO was also prepared by the method above. Results are summarized in Table 5.

Table 5

Yields and extraction ratios of Tc-99m complexes with EnAO and PnAO.

| | Percent Yield[a] | Octanol/buffer[b] Extraction Ratio | $CHCl_3$/buffer[b] Extraction Ratio |
|---|---|---|---|
| Tc-99m-EnAO | >95% | | 152 |
| Tc-99m-PnAO | >95% | 55 | 153 |

[a]Prepared as described above at pH 8.1 and ligand concentrations ranging from $10^{-4}$ to 5 x $10^{-2}$M.
[b]buffer - pH 7.4 bicarbonate buffer at a concentration of 0.025M.

Structure of Solid Tc-PnAO with Tc-99

Structure of solid Tc-PnAO was determined by x-ray diffraction using data collected on an Enraf-Nonius CAD4 diffractometer. The structure solution was accomplished using the SDP program package of Enraf-Nonius on a PDP11/34 computer and has been refined to a current agreement factor of 2.8%.

The structure described above is consistent with the chemical formula $TcC_{13}H_{25}N_4O_3$ or $[TcO(PnAO-3H)]^{\circ}$ where the −3H represents the loss of three H atoms from the PnAO ligand, one from an oxime oxygen and two from the amine nitrogens. The Tc is in the +5 oxidation state resulting in a net charge of zero for the ligand.

A sample of the solid was dissolved in $CH_3OH$ and subjected to electrophoresis and paper chromatography. The results indicated a complex yield of greater than 95%, consistent with that of the complexes formed with Tc-99m.

Example 20

Preparation of Tc-99m complexes of EnAO, PnAO and PnAO derivatives

The following general method was used to prepare Tc-99m complexes. The first step involves the preparation of a solution of the ligand within a pH range of 7.5 to 8.5. Both the physical form and the nature of the ligand will influence the preferred method for the preparation of the solution.

i) *The ligand as a water soluble salt* (for example, the mono, di, or trihydrochloride salt). 2 to 3 mg of the ligand is dissolved in 0.5 ml of saline, and the pH of the solution adjusted to be within the desired range by the addition of 0.5 ml of 0.02M sodium bicarbonate solution in saline.

ii) *A water soluble free base*. Approximately 2 mg of the ligand is dissolved in 0.5 ml of $10^{-3}$M HCl, and the pH of the solution adjusted to 7.5 and 8.5 by the addition of 0.5 ml 0.02M sodium bicarbonate solution in saline.

iii) *A free base or salt of the ligand with poor water solubility*. 2 to 3 mg of the ligand is dissolved in 0.5 ml of ethanol and 0.5 ml of 0.02M sodium bicarbonate solution in saline is added to adjust the pH to the required level.

To the solution of the ligand at the required pH is added 0.2 ml of a saturated solution of stannous tartrate in saline and 0.5 to 1.5 ml of Tc-99m pertechnetate, obtained from a Mo-99/Tc-99m generator system. Analysis of the resultant mixture indicated that reduction of pertechnetate (to a lower oxidation form of technetium), and complexation of the reduced technetium to the ligand is complete after standing at ambient temperature for 10 minutes.

Analysis of the Tc-99m complexes

1. *Thin Layer Chromatography*

Glass fibre strips impregnated with silica gel form the stationary phase of a fast and accurate analytical system for the Tc-99m complexes of EnAO, PnAO and PnAO derivatives. Two strips, each measuring 20 cm × 2 cm were used in each analysis. Approximately five microlitres of the solution containing the complex was applied 1 cm from the base of each strip, and one strip developed with saline, the other with ethylmethylketone (MEK).

Determination of the distribution of radioactivity along each strip was conducted by means of a 100 channel analysis system interfaced to a Nova computer, programmed for peak integration. The Table, below, indicates the RF values of the major components of the Tc-99m solutions. With all ligands, the observed radiochemical purity of the Tc-99m complex was greater than 80%, and generally greater than 95%.

Silica gel on glass fibre chromatography

| | Observed RF values | |
|---|---|---|
| Eluent: | MEK | Saline |
| Tc-99m colloid | Ø | Ø |
| Tc-99m pertechnetate | 1.0 | 1.0 |
| Tc-99m complex | 0.0.2 | 0.9—1.0 |

2. *HPLC analysis*

HPLC analysis was conducted on a 150 × 4.1 mm stainless steel column packed with a divinylbenzenestyrene copolymer, reverse phase resin. The column was connected to a commercial dual pump gradient chromatographic system. The radioactivity in the eluent was detected by a NaI scintillation detector connected to a ratemeter. Output from the ratemeter was directed to a chart recorder and microcomputer programmed for peak integration.

Two solvents were used HPLC analysis; 0.02M $NaH_2PO_4$ in water, adjusted to pH 7.0, and containing 2% methanol (Solvent A) and HPLC grade THF (Solvent B). Two gradient solvent elution systems were developed, as follows:

*System 1*

A flow of 100% solvent A was established at a flow rate of 2 ml/min prior to injection of a 10 microlitre sample. At the point of injection a linear solvent gradient was introduced, which increased the proportion of solvent B to 25% at six minutes post injection. The proportion of solvent B was maintained at 25% for a further 12 minutes; then reduced to 0% over 2 minutes.

*System 2*

A flow of 100% solvent A at a flow rate of 2 ml/min was established prior to injection of a 10 microlitre sample. At the point of injection, a linear solvent gradient was introduced which increased the proportion of solvent B to 75% over 13 minutes. The proportion of solvent B was maintained for a further 5 minutes, then reduced to 0% over 3 minutes.

System 1 was used in all cases, except those in which the high lipophilicity of the Tc-99m complex necessitated the higher organic solvent content of system 2 to enable elution of the complex from the column. Table 6 below lists the observed retention times of radioactive components.

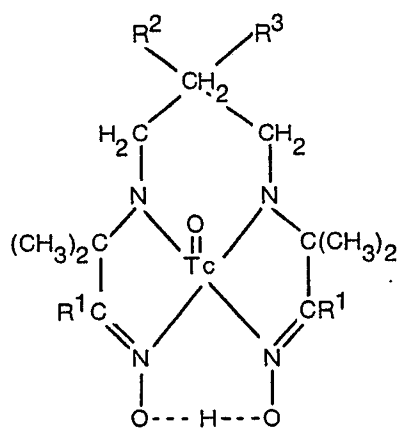

Table 6

| Tc-99m, complex | | | Elution System | Observed retention time (minutes) |
|---|---|---|---|---|
| $R^1$ | $R^2$ | $R^3$ | | |
| $CH_3$ | H | H | 1 | 12.6 |
| $CH_3$ | H | H | 2 | 9.9 |
| Et | H | H | 2 | 12.3 |
| n-butyl | H | H | 2 | 14.5 |
| n-nonyl | H | H | 2 | 16.5 |
| $CH_3$ | morpholinyl | H | 1 | 9.0 |
| $CH_3$ | hydroxyl | H | 1 | 9.1 |
| $CH_3$ | piperadinyl | H | 1 | 11.0 |
| $CH_3$ | morpholinyl-ethyl | H | 1 | 9.6 |

When the ligand (V) of Example 16 was complexed with Tc-99m a complexation yield of 90 ± 5% was obtained and a single hydrophobic peak was observed during HPLC analysis. The retention volume of this Tc-99m-n-pentyl-PnAO derivative is larger than that of Tc-99m-PnAO using the gradient elution HPLC method, indicating that this derivative, as expected, has a higher hydrophobicity than Tc-99m-PnAO.

Example 21

Preparation of Tc-99m-PnAO by ligand exchange

*Tc-99m-Citrate* was made by reducing 0.1 ml Tc-99m-pertechnetate in 1 ml 0.5*M* citrate at pH 6.5 with 0.1 ml stannous tartrate (saturated aqueous solution). The % chelate formed was >98%.

*Tc-99m-oxlate* was made by reducing 0.1 ml Tc-99m-pertechnetate in 1 ml 0.5*M* oxalate at pH 6.5 with 0.2 ml stannous tartrate (saturated aqueous solution). The % chelate formed was 85%. Less than 1% was in the pertechnetate form (determined using acetone solvent with paper chromatog) and 14.7% was Hydrolyzed-Red-Tc-99m (determined using saline as the eluant by paper chromatog).

Formation of Tc-99m-PnAO by ligand exchange was accomplished by:

(1) the addition of 0.1 ml of the Tc-99m-citrate solution to 1 ml of a 0.01*M* bicarbonate buffered saline solution at pH 9 containing 1 mg PnAO. The sample was mixed and allowed to stand for 20 minutes before injection into the HPLC. After 20 minutes, 91.3% of the Tc-99m activity was associated with PnAO as the Tc-99m-PnAO complex. Approximately 8.5% remained as Tc-99m-citrate.

(2) the addition of 0.1 ml of the Tc-99m-oxalate solution to 1 ml of a 0.01*M* bicarbonate buffered saline solution at pH 9 containing 1 mg PnAO. The sample was mixed and allowed to stand for 20 minutes. The % Tc-99m-activity associated with Tc-99m-PnAO was 85%, no activity remained as the Tc-99m-oxalate complex and 15% remained (unaffected) as the hydrolyzed-reduced-Tc-99m-form.

Examples 22 to 27 describe *in vivo* properties of the complexes prepared and characterized in the preceding Examples.

Example 22

In-vivo biodistribution studies of Tc-99m PnAO and derivatives of PnAO

0.1 ml of the Tc-99m complex solution is administered by intravenous injection (lateral tail vein) to each of 5 rats (140—220 g). The injected dose is equivalent to approximately 200 μCi of Tc-99m. Three rats are sacrificed at 2 minutes post injection, and two rats two hours post injection. At dissection the organs and tissue samples shown in the following Table are taken, and assayed for radioactivity. The uptake in each organ or tissue is calculated as a percentage of total activity recovered. Results are reported in Table 7. The Tc-99m EnAO demonstrates 0.85% i.d. uptake in rat brain at 15 seconds post injection.

Table-7

Biodistribution of Tc-PnAO derivatives in rats

2 minute sacrifice.

| Complex with compound of Example | % i.d. - average of 3 rats Blood | Lung | Liver | Heart | Brain |
|---|---|---|---|---|---|
| 2 | 5.26 | 1.39 | 35.9 | .13 | .67 |
| 3 | 8.42 | 2.17 | 23.7 | .51 | .85 |
| 4 | 4.22 | 1.34 | 25.5 | .88 | 1.22 |
| 5 | 28.6 | 7.95 | 55.63 | .25 | .20 |
| 6 | 13.63 | 1.43 | 21.53 | .48 | 1.18 |
| 7 | 11.07 | 1.12 | 20.16 | .27 | .20 |
| 8 | 7.75 | 1.01 | 20.73 | .45 | 0.17 |
| 9 | 7.74 | 1.30 | 16.24 | .36 | .12 |
| 10 | 5.57 | 1.48 | 16.93 | .33 | .13 |
| 11 | 15.5 | 4.6 | 38.2 | .91 | .11 |
| 12 | 14.1 | 44.7 | 32.2 | .56 | .07 |
| 13 | 8.0 | 1.24 | 28.8 | .53 | .72 |
| 14 | 7.8 | 1.7 | 34.9 | .87 | .27 |
| 15 | 11.2 | 2.9 | 14.9 | .68 | 1.5 |

Table-7 (continued)

Biodistribution of Tc-PnAO derivatives in rats

2 hour sacrifice.

| Complex with compound of Example | Blood | Lung | Liver | Heart | Brain | GI Tract |
|---|---|---|---|---|---|---|
| | % i.d. - average of 2 rats | | | | | |
| 2 | 4.6 | .41 | 14.2 | .11 | .05 | 59.5 |
| 3 | 6.5 | .92 | 21.5 | .18 | .08 | 41.0 |
| 4 | 3.6 | .41 | 14.8 | .10 | .06 | 53.4 |
| 5 | 10.8 | 3.3 | 61.8 | .40 | .05 | 8.6 |
| 6 | 4.2 | .64 | 23.1 | .25 | .11 | 37.2 |
| 7 | 4.4 | .33 | 4.9 | .10 | .03 | 42.0 |
| 8 | 2.1 | .36 | 7.5 | .14 | .05 | 51.3 |
| 9 | 1.8 | .34 | 10.5 | .11 | .03 | 44.3 |
| 10 | 0.9 | 0.5 | 16.8 | .14 | .03 | 47.5 |
| 11 | 5.2 | .77 | 17.6 | .18 | .03 | 41.0 |
| 12 | 4.6 | 14.1 | 36.1 | .56 | .03 | 14.5 |
| 13 | 5.0 | .45 | 21.3 | .15 | .05 | 48.5 |
| 14 | 1.2 | .21 | 20.1 | .05 | .01 | 61.5 |
| 15 | 5.4 | 2.1 | 11.3 | .46 | 1.1 | 17.0 |

Example 23

In-vivo stability of Tc-99m PnAO

Following iv administration of Tc-99m PnAO in 300—350 g anaesthetised rats, bile and urine samples were collected and analysed by HPLC. Bile samples were collected for 30 minutes via a cunnula placed in the common bile duct. In all cases, >90% of the Tc-99m activity present in these samples was the PnAO complex, suggesting good stability of the complex in the body and body fluids.

Example 24

Uptake of Tc-99m in red blood cells

Following incubation of a 0.1 ml sample of Tc-99m PnAO with a sample of whole blood for five minutes, and separation of the red cells (RBC) it was observed that 64.9 ± 1.9% of the activity was localised in the RBC fraction. Removal of the RBCs from plasma, and resuspension of the cells in saline allowed reequilibration of the activity taken up by the cells. After 5 minutes, the RBC/saline ratio was 2.36. A second saline wash of the RBCs gave a ratio of 2.81.

These data indicate that Tc-99m PnAO will diffuse freely across cellular walls.

Example 25

Brain extraction efficiency of Tc-99m PnAO

The technique published by Raichle et al. (Am. J. Physiol. 1976, 230, 543—552) was used to estimate the extraction efficiency of Tc-99m PnAO in brain tissue.

The apparatus consisted of a single gamma ray detector, fitted with a ⅛ inch single hole collimator, and connected to a multi-channel analyser operated on the multiscalar mode. The anaesthetised animal was position on the probe so that the brain was directly over the hole of the collimator. A bolus injection of Tc-99m PnAO was made into the internal carotid artery. The bolus was delivered in <0.5 seconds through a 30 gauge needle, so as not to impede blood flow. The activity per 0:1 second counted by the detector was plotted as a function of time for ten minutes. The extraction efficiency determined by this method in rats, rabbits, and monkeys was observed in the range 70—90%.

Example 26

Toxicity Studies with PnAO

A solution consisting of 4 mg of PnAO/ml of physiological saline at pH 8.1 (0.01M $HCO_3$ buffer) was prepared for injection. Fifty microlitres was injected into the tail vein of each of the 10 unanaesthetised Swiss-Webster mice weighing between 18—22 gms (corresponding to a dose in mg PnAO/kg body weight that is 1000—1500 times the anticipated dose to be administered to humans for diagnostic radionuclidic studies). A group of 10 Swiss-Webster mice in the same weight range were similarly injected with 50 microlitres of physiological saline. Both groups were housed in the same animal quarters for 24 days, and the variation in body weight recorded 4 times each week. No significant difference was observed between the group which received the PnAO composition and the control group. No visual effects (eg., seizures, abnormal behaviour, etc.) were observed in any of the animals during or at any time after injection of either group. After observation for 24 days, no gross abnormality was observed in any of the organs taken after sacrifice. Thus, it is understood that the toxicity of PnAO is extremely low.

Example 27

Protocol for Clinical Studies with Tc-99m PnAO

Prior to the study, a blood sample is taken from the patient, and temperature, pulse rate, respiration rate and blood pressure are measured. The patient is placed in a supine position and a gamma camera positioned to obtain a vertex view. The gamma camera is connected to an image processing computer.

Following i.v. administration of 15 mCi of a solution of Tc-99m PnAO dynamic data is stored by the computer for ten minutes. In addition a scintiphotograph is taken of the gamma camera image 30 seconds—90 seconds post injection. Following the study, digitised images are obtained corresponding to several time points after administration of the radiopharmaceutical. In stroke patients, the affected areas of the brain can be observed by decreased uptake of the radiopharmaceutical in images corresponding to 30—120 seconds post injection. The results show good agreement with images obtained by computer tomography.

At 15 minute intervals the patient's temperature, blood pressure, pulse rate and respiration are monitored, and at one hour post injection, a further blood sample is taken. No changes or side-effects of the radiopharmaceutical were observed.

**Claims**

1. A lipophilic macrocyclic complex of Technetium-99m useful as a diagnostic radiopharmaceutical which can be formed by complexing in aqueous solution Tc-99m pertechnetate under reducing conditions with an alkylene amine oxime containing 2 or 3 carbon atoms in the alkylene group, which group is unsubstituted or substituted, the complex having a core with a zero net charge, containing an O—H—O ring closure bond, and being sufficiently stable for parenteral administration and imaging by scintillation

scanning, any alkylene substituents present being of the kind useful for adapting radionuclide ligands tor body imaging applications, which complex has the formula

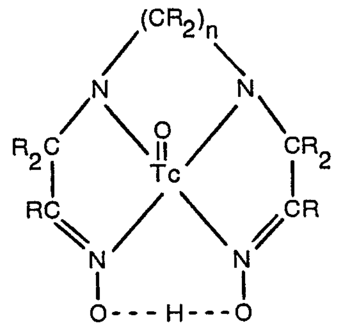

(2)

where n is 2 or 3, and the groups R may be the same or different and each is hydrogen, hydrocarbon of up to 22 carbon atoms which may be alkyl, alkenyl, alkaryl, aralkyl or aryl, primary secondary or tertiary amine, primary, secondary or tertiary amide, carboxylic acid or carboxylic acid ester, hydroxyl or alkoxyl, or an acceptor-acid group, and may be substituted or unsubstituted.

2. A complex as claimed in claim 1, wherein the technetium is present in the 5+ valency.

3. A complex as claimed in claim 1 or claim 2, which has the formula

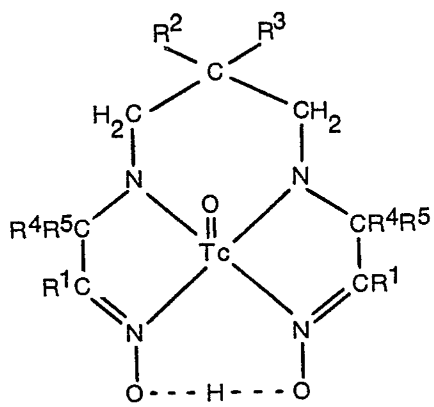

(3)

where each $R^1$, $R^4$ and $R^5$ is hydrogen or C1 to C12 alkyl, and each of $R^2$ and $R^3$ is hydrogen, hydroxyl, C1 to C12 alkoxyl, an up to C22 hydrocarbon which may be alkyl, alkenyl, alkaryl, aralkyl or aryl, or tertiary amine with 1 to 20 carbon atoms, or $R^2$ and $R^3$ form, together with the carbon atom to which they are attached, a cycloaliphatic group which may be amine substituted.

4. A complex as claimed in claim 3, wherein each of $R^2$ and $R^3$ is hydrogen or C1 to C4 alkyl.

5. A complex as claimed in claim 1, which is the technetium-99m complex of 4,7-diazo-3,3,8,8-tetramethyldecane-2,9-dione bisoxime.

6. A complex as claimed in claim 1, which is the technetium-99m complex of 4,8,diaza-3,3,9,9-tetramethylundecane-2,10-dione bisoxime.

7. A complex as claimed in claim 1, which is the technetium-99m complex of 4,8-diaza-3,3,6,6,9,9-hexamethylundecane-2,10-dione bisoxime.

8. A complex as claimed in any one of claims 1 to 7, which has a partition coefficient between lipid and water and a molecular weight such that *in vivo* it is capable of diffusing across the blood brain barrier and of being retained in the brain for a time sufficient for it to be used for diagnostic purposes.

9. A complex as claimed in any one of claims 1 to 7, which complex has the property of penetrating cellular membranes and localizing in lung tissue.

10. A complex as claimed in any one of claims 1 to 7, which complex has the property of localizing in myocardial tissue.

**Patentansprüche**

1. Lipophiler makrozyklischer Komplex von Technetium-99m, verwendbar als diagnostisches Radiopharmazeutikum, der gebildet werden kann durch Komplexieren von Tc-99m-Pertechnetat in wäßriger Lösung unter reduzierenden Bedingungen mit einem Alkylenaminoxim, enthaltend 2 oder 3 Kohlenstoffatome in der Alkylengruppe, die nicht-substituiert oder substituiert sein kann, wobei der Komplex einen Kern mit einer Null-Nettoladung hat, eine O—H—O geschlossene Ringbindung enthält und

ausreichend stabil ist für eine parenterale Verabreichung und Darstellung durch Scintillationsabtasten, wobei jeder anwesende Alkylensubstituent derart ist, daß er zur Anpassung von Radionukleid-Liganden für Körper-darstellende Verwendungen geeignet ist und der Komplex die Formel

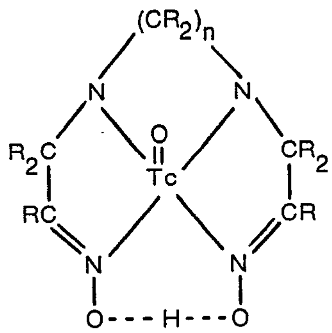

, (2)

hat, worin n 2 oder 3 ist und die Gruppen R gleich oder unterschiedlich sein können und jede Wasserstoff, Kohlenwasserstoff von bis zu 22 Kohlenstoffatomen, die Alkyl, Alkenyl, Alkaryl, Aralkyl oder Aryl, primäres, sekundäres oder tertiäres Amin, primäres, sekundäres oder tertiäres Amid, Carbonsäure oder Carbonsäureester, Hydroxyl oder Alkoxyl oder eine Akkzeptor-Säuregruppe sein und substituiert oder nichtsubstituiert sein kann.

2. Komplex nach Anspruch 1, worin das Technetium in der 5+ Wertigkeit vorliegt.

3. Komplex nach Anspruch 1 oder 2, der Formel

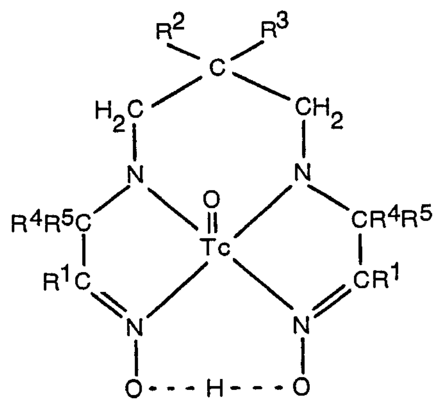

(3)

worin jedes $R^1$, $R^4$ und $R^5$ Wasserstoff oder ein C1 bis C12-Alkyl und jedes $R^2$ und $R^3$ Wasserstoff, Hydroxyl, ein C1 bis C12-Alkoxyl und bis zu C22 Kohlenwasserstoff ist, der Alkyl, Alkenyl, Alkaryl, Aralkyl oder Aryl oder tertiäres Amin mit 1 bis 20 Kohlenstoffatomen sein kann, oder worin $R^2$ und $R^3$ zusammen mit dem Kohlenstoffatom, an das sie angebunden sind, eine zykloaliphatische Gruppe bilden, die Amin-substituiert sein kann.

4. Komplex nach Anspruch 3, worin jedes $R^2$ und $R^3$ Wasserstoff oder ein C1 bis C4-Alkyl ist.

5. Komplex nach Anspruch 1, der der Technetium-99m-Komplex von 4,7,Diazo-3,3,8,8-tetramethyl-decan-2,9-dion Bisoxim ist.

6. Komplex nach Anspruch 1, der der Technetium-99m-Komplex von 4,8,Diaza-3,3,9,9-tetramethylun-decan-2,10-dion Bisoxim ist.

7. Komplex nach Anspruch 1, der der Technetium-99m-Komplex von 4,8,Diaza-3,3,6,6,9,9-hexamethyl-undecan-2,10-dion Bisoxim ist.

8. Komplex nach einem der Ansprüche 1 bis 7, der einen Verteilungskoeffizienten zwischen Lipid und Wasser und ein solches Molekulargewicht hat, daß er *in vivo* fähig ist die Blut-Gehirn-Schranke zu durchdringen und im Gehirn so lange gehalten werden kann, um für diagnostische Zwecke verwendet werden zu können.

9. Komplex nach einem der Ansprüche 1 bis 7, der die Eigenschaft hat in Zellmembrane einzudringen und in Lungengewebe lokalisiert werden kann.

10. Komplex nach einem der Ansprüche 1 bis 7, der in Myokardgewebe lokalisiert werden kann.

**Revendications**

1. Un complexe macrocyclique liphophile de technétium-99m comme agent de radiodiagnostic, qui peut être formé par complexage en solution aqueuse d'un pertechnétate de Tc-99m en milieu réducteur, avec une oxime d'une alkylène-diamine à deux ou trois atomes de carbone, avec ou sans substituants sur

le groupe alkylène, complexe qui a un centre à charge globale nulle, comporte une liaison de cyclisation O—H—O et reste suffisamment stable pour des administrations par la voie parentérale et des visualisations par balayage de scintillation, tous substituants du groupe alkylène éventuellement présents permettant d'adapter des coordinats de radionucléides à des applications d'imagerie médicale, complexe dont la formule est la suivante:

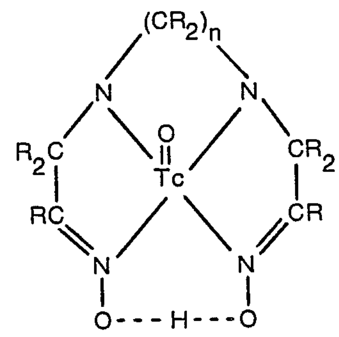

(2)

formule dans laquelle n est le nombre 2 ou 3 et les R, qui peuvent être identiques ou différents les uns des autres, sont chacun l'hydrogène, un radical hydrocarboné pouvant avoir jusqu'à 22 atomes de carbone et qui peut être un alkyle, un alcényle, un alcaryle, un aralkyle ou un aryle, ou encore un groupe amino ou amido primaire, secondaire ou tertiaire, un groupe carboxylique ou un ester de ce groupe, un groupe hydroxy ou alcoxy ou un groupe accepteur d'acides, avec ou sans substituants.

2. Un complexe selon la revendication 1 dans lequel le technétium est à l'état de valence 5+.

3. Un complexe selon la revendication 1 ou 2, de formule:

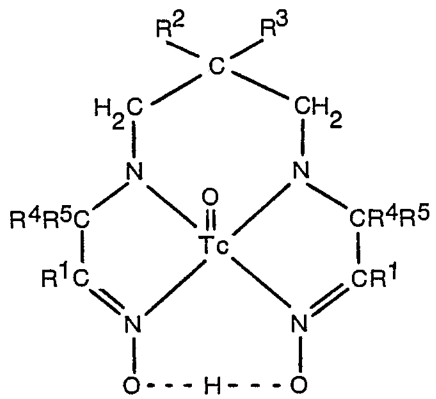

(3)

dans laquelle $R^1$, $R^4$ et $R^5$ sont chacun l'hydrogène ou un alkyle en $C_1$ à $C_{12}$ et $R^2$ et $R^3$ sont chacun l'hydrogène, un hydroxyle, un alcoxy en $C_1$ à $C_{12}$, un radical hydrocarboné pouvant avoir jusqu'à 22 atomes de carbone et qui peut être un alkyle, un alcényle, un alcaryle, un aralkyle ou un aryle, ou bien un groupe amino tertiaire de 1 à 20 atomes de carbone, ou encore $R^2$ et $R^3$ peuvent former, ensemble et avec l'atome de carbone auquel ils sont liés, un groupe cycloaliphatique éventuellement aminé.

4. Un complexe selon la revendication 3 dans lequel $R^2$ et $R^3$ sont chacun l'hydrogène ou un alkyle en $C_1$ à $C_4$.

5. Un complexe selon la revendication 1 qui est le complexe de technétium-99m avec la bisoxime de la 4,7-diaza-3,3,8,8-tétraméthyldécane-2,9-dione.

6. Un complexe selon la revendication 1 qui est le complexe de technétium-99m avec la bisoxime de la 4,8-diaza-3,3,9,9-tétraméthylundécane-2,10-dione.

7. Un complexe selon la revendication 1 qui est le complexe de technétium-99m avec la bisoxime de la 4,8-diaza-3,3,6,6,9,9-hexaméthylundécane-2,10-dione.

8. Un complexe selon l'une quelconque des revendications 1 à 7 qui a un coefficient de partage entre lipides et eau et une masse moléculaire lui permettant in vivo de diffuser à travers la barrière sang-cerveau et d'être retenu dans le cerveau pendant un temps suffisant pour pouvoir faire un diagnostic.

9. Un complexe selon l'une quelconque des revendications 1 à 7 qui peut pénétrer les membranes cellulaires et se localiser dans le tissu pulmonaire.

10. Un complexe selon l'une quelconque des revendications 1 à 7 qui peut se localiser dans le tissu du myocarde.